# EUROPEAN PATENT SPECIFICATION

(11) **EP 4 401 081 B1**
(45) Date of publication and mention of the grant of the patent: **03.09.2025**
(21) Application number: 24175027.2
(22) Date of filing: 23.07.2020
(51) Int. Cl.: G06F 16/28, G16B 50/00, G16B 10/00

(54) **CLUSTERING OF MATCHED SEGMENTS TO DETERMINE LINKAGE OF DATASET IN A DATABASE**
GRUPPIERUNG VON ANGEPASSTEN SEGMENTEN ZUR BESTIMMUNG DER VERKNÜPFUNG EINES DATENSATZES IN EINER DATENBANK
REGROUPEMENT DE SEGMENTS ADAPTÉS POUR DÉTERMINER LA LIAISON D'UN ENSEMBLE DE DONNÉES DANS UNE BASE DE DONNÉES

(30) Priority: 02.08.2019 US 201962882188 P
(43) Date of publication of application: 17.07.2024
(62) Divisional of application: 20849090.4
(73) Proprietor: Ancestry.com DNA, LLC, Lehi, UT 84043 (US)
(72) Inventor: NGUYEN, Thi Hong Luong, Utah 84043 (US)
(74) Representative: Gill Jennings & Every LLP

(56) References cited:
- US-A1- 2017 213 127
- CHRISTIAN ALLENDE ET AL: "Treelink: data integration, clustering and visualization of phylogenetic trees", BMC BIOINFORMATICS, BIOMED CENTRAL LTD, LONDON, UK, vol. 16, no. 1, 29 December 2015 (2015-12-29), pages 1 - 6, XP021237444, DOI: 10.1186/S12859-015-0860-1

## Description

### CROSS-REFERENCE TO RELATED APPLICATION

The present application claims the benefit of U.S. Provisional Patent Application No. 62/882,188 filed on August 2, 2019.

### FIELD

The technical field of the invention relates to performing haplotype phasing and resolving conflicting genotype datasets.

### BACKGROUND

A large-scale database such as user profile and genetic database can include billions of data records. This type of database may allow users to build their family trees, research their family history, and make meaningful discoveries about the lives of their ancestors. Users may try to identify relatives with datasets in the database. However, identifying relatives in the sheer amount of data is not a trivial task. Datasets associated with different individuals may not be connected without a proper determination of how the datasets are related. Comparing a large number of datasets without a concrete strategy may also be computational infeasible because each dataset may also include a large number of data bits.

US2017/213127A1 is related to building a family tree loading sets of DNA matches, creating clusters of overlapping DNAs and linking clusters of ancestors.

### SUMMARY

Disclosed herein relates to example embodiments that link datasets in a database. In one embodiment, a computer-implemented method for linking individuals' datasets in a database is described. The computer-implemented method may include receiving a target individual dataset of a target individual and a plurality of additional individual datasets. The computer-implemented method may also include generating a plurality of sub-cluster pairs of first parental groups and second parental groups. At least one of sub-cluster pairs includes a first parental group that includes a first set of matched segments selected from the plurality of additional individual datasets and a second parental group that includes a second set of matched segments selected from the plurality of additional individual datasets. The computer-implemented method may further include linking the first parental groups and the second parental groups across the plurality of sub-cluster pairs to generate at least one super-cluster of a parental side. The computer-implemented method may further include assigning metadata to one or more additional individual datasets of the plurality of additional individual datasets. The metadata may specify that the one or more additional individual datasets are connected to the target individual dataset by the parental side of the super-cluster.

In yet another embodiment, a non-transitory computer readable medium that is configured to store instructions is described. The instructions, when executed by one or more processors, cause the one or more processors to perform a process that includes steps described in the above computer-implemented methods or described in any embodiments of this disclosure. In yet another embodiment, a system may include one or more processors and a storage medium that is configured to store instructions. The instructions, when executed by one or more processors, cause the one or more processors to perform a process that includes steps described in the above computer-implemented methods or described in any embodiments of this disclosure.

The invention is defined by the features of the independent claims. Further embodiments are the subject-matter of the dependent claims.

### BRIEF DESCRIPTION OF THE DRAWINGS

Figure (FIG.) 1 illustrates a diagram of a system environment of an example computing system, in accordance with some embodiments.
FIG. 2 is a block diagram of an architecture of an example computing system, in accordance with some embodiments.
FIG. 3 is a flowchart depicting an example process for generating match clusters for identifying parental lineages of ancestors or relatives of a target individual, in accordance with some embodiments.
FIGS. 4A, 4B, 4C, and 4D are conceptual diagrams comparing segments of DNA data between two or more potential relatives and a target individual, in accordance with some embodiments.
FIG. 4E is a flowchart depicting an example haplotype phasing and genotype imputation process, in accordance with some embodiments.
FIG. 5 is a flowchart depicting an example process for linking sub-clusters to generate super-clusters, in accordance with some embodiments.
FIG. 6 is an example flowchart depicting a process for generating one or more super-clusters and their linking result using a bipartite graph by applying forward formulation, in accordance with some embodiments.
FIG. 7 is a conceptual diagram of an example initial bipartite graph, in accordance with some embodiments.
FIG. 8 is a conceptual diagram of an example bipartite graph with added edges, in accordance with some embodiments.
FIG. 9 is an example flowchart depicting a process for generating one or more super-clusters and their linking result using a bipartite graph by applying backward formulation, in accordance with some embodiments.
FIG. 10 is a conceptual diagram of an example bipartite graph with removed edges, in accordance with some embodiments.
FIG. 11 is a block diagram of an example computing device, in accordance with some embodiments.

The figures depict various embodiments for purposes of illustration only. One skilled in the art will readily recognize from the following discussion that alternative embodiments of the structures and methods illustrated herein may be employed without departing from the principles described herein.

### DETAILED DESCRIPTION

The figures (FIGs.) and the following description relate to preferred embodiments by way of illustration only. One of skill in the art may recognize alternative embodiments of the structures and methods disclosed herein as viable alternatives that may be employed without departing from the principles of what is disclosed.

Reference will now be made in detail to several embodiments, examples of which are illustrated in the accompanying figures. It is noted that wherever practicable similar or like reference numbers may be used in the figures and may indicate similar or like functionality. The figures depict embodiments of the disclosed system (or method) for purposes of illustration only. One skilled in the art will readily recognize from the following description that alternative embodiments of the structures and methods illustrated herein may be employed without departing from the principles described herein.

### EXAMPLE SYSTEM ENVIRONMENT

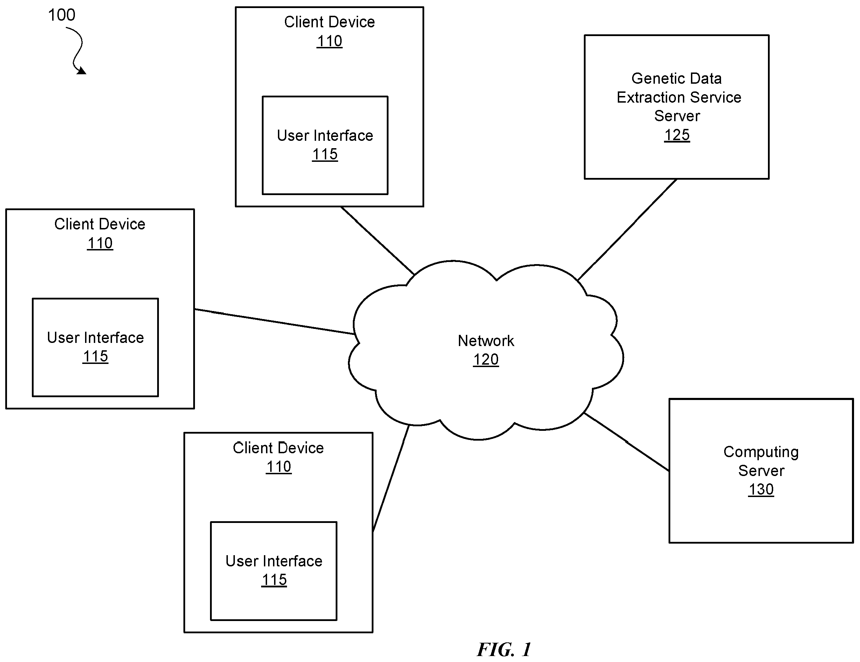

FIG. 1 illustrates a diagram of a system environment 100 of an example computing server 130, in accordance with some embodiments. The system environment 100 shown in FIG. 1 includes one or more client devices 110, a network 120, a genetic data extraction service server 125, and a computing server 130. In various embodiments, the system environment 100 may include fewer or additional components. The system environment 100 may also include different components.

The client devices 110 are one or more computing devices capable of receiving user input as well as transmitting and/or receiving data via a network 120. Example computing devices include desktop computers, laptop computers, personal digital assistants (PDAs), smartphones, tablets, wearable electronic devices (e.g., smartwatches), smart household appliance (e.g., smart televisions, smart speakers, smart home hubs), Internet of Things (IoT) devices or other suitable electronic devices. A client device 110 communicates to other components via the network 120. Users may be customers of the computing server 130 or any individuals who access the system of the computing server 130, such as an online website or a mobile application. In some embodiments, a client device 110 executes an application that launches a graphical user interface (GUI) for a user of the client device 110 to interact with the computing server 130. The GUI may be an example of a user interface 115. A client device 110 may also execute a web browser application to enable interactions between the client device 110 and the computing server 130 via the network 120. In another embodiment, the user interface 115 may take the form of a software application published by the computing server 130 and installed on the user device 110. In yet another embodiment, a client device 110 interacts with the computing server 130 through an application programming interface (API) running on a native operating system of the client device 110, such as IOS or ANDROID.

The network 120 provides connections to the components of the system environment 100 through one or more sub-networks, which may include any combination of local area and/or wide area networks, using both wired and/or wireless communication systems. In some embodiments, a network 120 uses standard communications technologies and/or protocols. For example, a network 120 may include communication links using technologies such as Ethernet, 802.11, worldwide interoperability for microwave access (WiMAX), 3G, 4G, Long Term Evolution (LTE), 5G, code division multiple access (CDMA), digital subscriber line (DSL), etc. Examples of network protocols used for communicating via the network 120 include multiprotocol label switching (MPLS), transmission control protocol/Internet protocol (TCP/IP), hypertext transport protocol (HTTP), simple mail transfer protocol (SMTP), and file transfer protocol (FTP). Data exchanged over a network 120 may be represented using any suitable format, such as hypertext markup language (HTML) or extensible markup language (XML). In some embodiments, all or some of the communication links of a network 120 may be encrypted using any suitable technique or techniques such as secure sockets layer (SSL), transport layer security (TLS), virtual private networks (VPNs), Internet Protocol security (IPsec), etc. The network 120 also includes links and packet switching networks such as the Internet.

Individuals, who may be customers of a company operating the computing server 130, provide biological samples for analysis of their genetic data. Individuals may also be referred to as users. In some embodiments, an individual uses a sample collection kit to provide a biological sample (e.g., saliva, blood, hair, tissue) from which genetic data is extracted and determined according to nucleotide processing techniques such as amplification and sequencing. Amplification may include using polymerase chain reaction (PCR) to amplify segments of nucleotide samples. Sequencing may include sequencing of deoxyribonucleic acid (DNA) sequencing, ribonucleic acid (RNA) sequencing, etc. Suitable sequencing techniques may include Sanger sequencing and massively parallel sequencing such as various next-generation sequencing (NGS) techniques including whole genome sequencing, pyrosequencing, sequencing by synthesis, sequencing by ligation, and ion semiconductor sequencing. In some embodiments, a set of SNPs (e.g., 300,000) that are shared between different array platforms (e.g., Illumina OmniExpress Platform and Illumina HumanHap 650Y Platform) may be obtained as the genetic data. Genetic data extraction service server 125 receives biological samples from users of the computing server 130. The genetic data extraction service server 125 performs sequencing of the biological samples and determines the base pair sequences of the individuals. The genetic data extraction service server 125 generates the genetic data of the individuals based on the sequencing results. The genetic data may include data sequenced from DNA or RNA and may include base pairs from coding and/or noncoding regions of DNA.

The genetic data may take different forms and include information regarding various biomarkers of an individual. For example, in some embodiments, the genetic data may be the base pair sequence of an individual. The base pair sequence may include the whole genome or a part of the genome such as certain genetic loci of interest. In another embodiment, the genetic data extraction service server 125 may determine genotypes from sequencing results, for example by identifying genotype values of single nucleotide polymorphisms (SNPs) present within the DNA. The results in this example may include a sequence of genotypes corresponding to various SNP sites. A SNP site may also be referred to as a SNP loci. A genetic locus is a segment of a genetic sequence. A locus can be a single site or a longer stretch. The segment can be a single base long or multiple bases long. In some embodiments, the genetic data extraction service server 125 may perform data pre-processing of the genetic data to convert raw sequences of base pairs to sequences of genotypes at target SNP sites. Since a typical human genome may differ from a reference human genome at only several million SNP sites (as opposed to billions of base pairs in the whole genome), the genetic data extraction service server 125 may extract only the genotypes at a set of target SNP sites and transmit the extracted data to the computing server 130 as the genetic dataset of an individual. SNPs, base pair sequence, genotype, haplotype, RNA sequences, protein sequences, phenotypes are examples of biomarkers.

The computing server 130 performs various analyses of the genetic data, genealogical data, and users' survey responses to generate results regarding the phenotypes and genealogy of users of computing server 130. Depending on the embodiments, the computing server 130 may also be referring to as an online server, a personal genetic service server, a genealogy server, a family tree building server, and/or a social networking system. The computing server 130 receives genetic data from the genetic data extraction service server 125 and stores the genetic data in the data store of the computing server 130. The computing server 130 may analyze the data to generate results regarding the genetics or genealogy of users. The results regarding the genetics or genealogy of users may include the ethnicity compositions of users, paternal and maternal genetic analysis, identification or suggestion of potential family relatives, ancestor information, analyses of DNA data, potential or identified traits such as phenotypes of users (e.g., diseases, appearance traits, other genetic characteristics, and other non-genetic characteristics including social characteristics), etc. The computing server 130 may present or cause the user interface 115 to present the results to the users through a GUI displayed at the client device 110. The results may include graphical elements, textual information, data, charts, and other elements such as family trees.

In some embodiments, the computing server 130 also allows various users to create one or more genealogical profiles of the user. The genealogical profile may include a list of individuals (e.g., ancestors, relatives, friends, and other people of interest) who are added or selected by the user or suggested by the computing server 130 based on the genealogical records and/or genetic records. The user interface 115 controlled by or in communication with the computing server 130 may display the individuals in a list or as a family tree such as in the form of a pedigree chart. In some embodiments, subject to user's privacy setting and authorization, the computing server 130 may allow information generated from the user's genetic dataset to be linked to the user profile and to one or more of the family trees. The users may also authorize the computing server 130 to analyze their genetic dataset and allow their profiles to be discovered by other users.

### EXAMPLE COMPUTING SERVER ARCHITECTURE

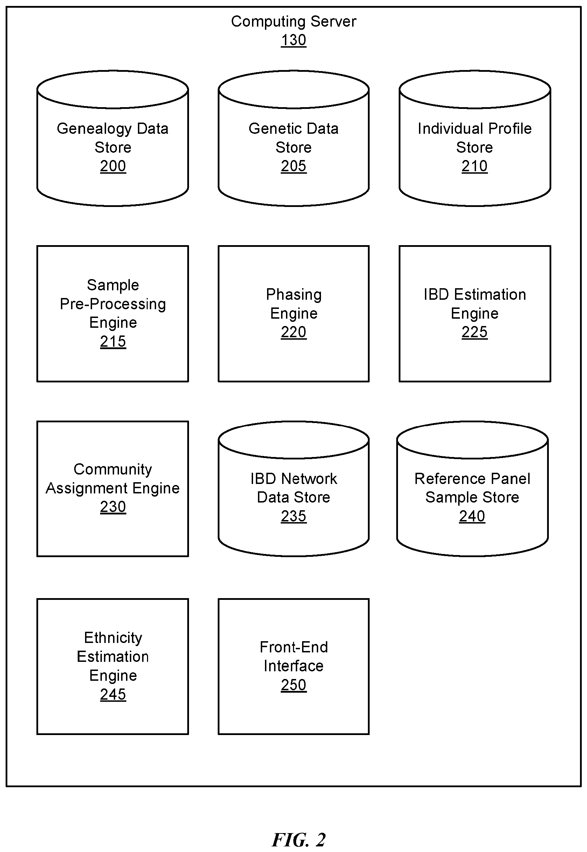

FIG. 2 is a block diagram of an architecture of an example computing server 130, in accordance with some embodiments. In the embodiment shown in FIG. 2, the computing server 130 includes a genealogy data store 200, a genetic data store 205, an individual profile store 210, a sample pre-processing engine 215, a phasing engine 220, an identity by descent (IBD) estimation engine 225, a community assignment engine 230, an IBD network data store 235, a reference panel sample store 240, an ethnicity estimation engine 245, and a front-end interface 250. The functions of the computing server 130 may be distributed among the elements in a different manner than described. In various embodiments, the computing server 130 may include different components and fewer or additional components. Each of the various data stores may be a single storage device, a server controlling multiple storage devices, or a distributed network that is accessible through multiple nodes (e.g., a cloud storage system).

The computing server 130 stores various data of different individuals, including genetic data, genealogical data, and survey response data. The computing server 130 processes the genetic data of users to identify shared identity-by-descent (IBD) segments between individuals. The genealogical data and survey response data may be part of user profile data. The amount and type of user profile data stored for each user may vary based on the information of a user, which is provided by the user as she creates an account and profile at a system operated by the computing server 130 and continues to build her profile, family tree, and social network at the system and to link her profile with her genetic data. Users may provide data via the user interface 115 of a client device 110. Initially and as a user continues to build her genealogical profile, the user may be prompted to answer questions related to the basic information of the user (e.g., name, date of birth, birthplace, etc.) and later on more advanced questions that may be useful for obtaining additional genealogical data. The computing server 130 may also include survey questions regarding various traits of the users such as the users' phenotypes, characteristics, preferences, habits, lifestyle, environment, etc.

Genealogical data may be stored in the genealogical data store 200 and may include various types of data that are related to tracing family relatives of users. Examples of genealogical data include names (first, last, middle, suffixes), gender, birth locations, date of birth, date of death, marriage information, spouse's information kinships, family history, dates and places for life events (e.g., birth and death), other vital data, and the like. In some instances, family history can take the form of a pedigree of an individual (e.g., the recorded relationships in the family). The family tree information associated with an individual may include one or more specified nodes. Each node in the family tree represents the individual, an ancestor of the individual who might have passed down genetic material to the individual, and the individual's other relatives including siblings, cousins, offspring in some cases. Genealogical data may also include connections and relationships among users of the computing server 130. The information related to the connections among a user and her relatives that may be associated with a family tree may also be referred to as pedigree data or family tree data.

In addition to user-input data, genealogical data may also take other forms that are obtained from various sources such as public records and third-party data collectors. For example, genealogical records from public sources include birth records, marriage records, death records, census records, court records, probate records, adoption records, obituary records, etc. Likewise, genealogical data may include data from one or more of a pedigree of an individual, the Ancestry World Tree system, a Social Security Death Index database, the World Family Tree system, a birth certificate database, a death certificate database, a marriage certificate database, an adoption database, a draft registration database, a veterans database, a military database, a property records database, a census database, a voter registration database, a phone database, an address database, a newspaper database, an immigration database, a family history records database, a local history records database, a business registration database, a motor vehicle database, and the like.

Furthermore, the genealogical data store 200 may also include relationship information inferred from the genetic data stored in the genetic data store 205 and information received from the individuals. For example, the relationship information may indicate which individuals are genetically related, how they are related, how many generations back they share common ancestors, lengths and locations of IBD segments shared, which genetic communities an individual is a part of, variants carried by the individual, and the like.

The computing server 130 maintains genetic datasets of individuals in the genetic data store 205. A genetic dataset of an individual may be a digital dataset of nucleotide data (e.g., SNP data) and corresponding metadata. A genetic dataset may contain data of the whole or portions of an individual's genome. The genetic data store 205 may store a pointer to a location associated with the genealogical data store 200 associated with the individual. A genetic dataset may take different forms. In some embodiments, a genetic dataset may take the form of a base pair sequence of the sequencing result of an individual. A base pair sequence dataset may include the whole genome of the individual (e.g., obtained from a whole-genome sequencing) or some parts of the genome (e.g., genetic loci of interest).

In another embodiment, a genetic dataset may take the form of sequences of genetic markers. Examples of genetic markers may include target SNP loci (e.g., allele sites) filtered from the sequencing results. A SNP locus that is single base pair long may also be referred to a SNP site. A SNP locus may be associated with a unique identifier. The genetic dataset may be in a form of a diploid data that includes a sequencing of genotypes, such as genotypes at the target SNP loci, or the whole base pair sequence that includes genotypes at known SNP loci and other base pair sites that are not commonly associated with known SNPs. The diploid dataset may be referred to as a genotype dataset or a genotype sequence. Genotype may have a different meaning in various contexts. In one context, an individual's genotype may refer to a collection of diploid alleles of an individual. In other contexts, a genotype may be a pair of alleles present on two chromosomes for an individual at a given genetic marker such as a SNP site.

A genotype at a SNP site may include a pair of alleles. The pair of alleles may be homozygous (e.g., A-A or G-G) or heterozygous (e.g., A-T, C-T). Instead of storing the actual nucleotides, the genetic data store 205 may store genetic data that are converted to bits. For a given SNP site, oftentimes only two nucleotide alleles (instead of all 4) are observed. As such, a 2-bit number may represent a SNP site. For example, 00 may represent homozygous first alleles, 11 may represent homozygous second alleles, and 01 or 10 may represent heterozygous alleles. A separate library may store what nucleotide corresponds to the first allele and what nucleotide corresponds to the second allele at a given SNP site.

A diploid dataset may also be phased into two sets of haploid data, one corresponding to a first parent side and another corresponding to a second parent side. The phased datasets may be referred to as haplotype datasets or haplotype sequences. Similar to genotype, haplotype may have a different meaning in various contexts. In one context, a haplotype may also refer to a collection of alleles that corresponds to a genetic segment. In other contexts, a haplotype may refer to a specific allele at a SNP site. For example, a sequence of haplotypes may refer to a sequence of alleles of an individual that are inherited from a parent.

The individual profile store 210 stores profiles and related metadata associated with various individuals appeared in the computing server 130. A computing server 130 may use unique individual identifiers to identify various users and other non-users that might appear in other data sources such as ancestors or historical persons who appear in any family tree or genealogical database. A unique individual identifier may a hash of certain identification information of an individual, such as a user's account name, user's name, date of birth, location of birth, or any suitable combination of the information. The profile data related to an individual may be stored as metadata associated with an individual's profile. For example, the unique individual identifier and the metadata may be stored as a key-value pair using the unique individual identifier as a key.

An individual's profile data may include various kinds of information related to the individual. The metadata about the individual may include one or more pointer associating genetic datasets such as genotype and phased haplotype data of the individual that are saved in the genetic data store 205. The metadata about the individual may also individual information related to family trees and pedigree datasets that include the individual. The profile data may further include declarative information about the user that was authorized by the user to be shared and may also include information inferred by the computing server 130. Other examples of information stored in a user profile may include biographic, demographic, and other types of descriptive information such as work experience, educational history, gender, hobbies, or preferences, location and the like. In some embodiments, the user profile data may also include one or more photos of the users and photos of relatives (e.g., ancestors) of the users that are uploaded by the users. A user may authorize the computing server 130 to analyze one or more photos to extract information, such as user's or relative's appearance traits (e.g., blue eyes, curved hair, etc.), from the photos. The appearance traits and other information extracted from the photos may also be saved in the profile store. User profile data may also be obtained from other suitable sources, including historical records (e.g., records related to an ancestor), medical records, military records, photographs, other records indicating one or more traits, and other suitable recorded data.

For example, the computing server 130 may present various survey questions to its users from time to time. The responses to the survey questions may be stored at individual profile store 210. The survey questions may be related to various aspects of the users and the users' families. Some survey questions may be related to users' phenotypes, while other questions may be related to environmental factors of the users.

Survey questions may concern health or disease-related phenotypes, such as questions related to the presence or absence of genetic diseases or disorders, inheritable diseases or disorders, or other common diseases or disorders that have a family history as one of the risk factors, questions regarding any diagnosis of increased risk of any diseases or disorders, and questions concerning wellness-related issues such as a family history of obesity, family history of causes of death, etc. The diseases identified by the survey questions may be related to single-gene diseases or disorders that are caused by a single-nucleotide variant, an insertion, or a deletion. The diseases identified by the survey questions may also be multifactorial inheritance disorders that may be caused by a combination of environmental factors and genes. Examples of multifactorial inheritance disorders may include heart disease, Alzheimer's diseases, diabetes, cancer, and obesity. The computing server 130 may obtain data of a user's disease-related phenotypes from survey questions of the health history of the user and her family and also from health records uploaded by the user.

Survey questions also may be related to other types of phenotypes such as the appearance traits of the users. A survey regarding appearance traits and characteristics may include questions related to eye color, iris pattern, freckles, chin types, finger length, dimple chin, earlobe types, hair color, hair curl, skin pigmentation, susceptibility to skin burn, bitter taste, male baldness, baldness pattern, presence of unibrow, presence of wisdom teeth, height, and weight. A survey regarding other traits also may include questions related to users' taste and smell such as the ability to taste bitterness, asparagus smell, cilantro aversion, etc. A survey regarding traits may further include questions related to users' body conditions such as lactose tolerance, caffeine consumption, malaria resistance, norovirus resistance, muscle performance, alcohol flush, etc. Other survey questions regarding a person's physiological or psychological traits may include vitamin traits and sensory traits such as the ability to sense an asparagus metabolite. Traits may also be collected from historical records, electronic health records, and electronic medical records.

The computing server 130 also may present various survey questions related to the environmental factors of users. In this context, an environmental factor may be a factor that is not directly connected to the genetics of the users. Environmental factors may include users' preferences, habits, and lifestyle. For example, a survey regarding users' preferences may include questions related to things and activities that users like or dislike, such as types of music a user enjoys, dancing preference, party-going preference, certain sports that a user plays, video games preferences, etc. Other questions may be related to the users' diet preference such as like or dislike a certain type of food (e.g., ice cream, egg). A survey related to habits and lifestyle may include questions regarding smoking habits, alcohol consumption and frequency, daily exercise duration, sleeping habits (e.g., morning person versus night person), sleeping cycles and problems, hobbies, and travel preferences. Additional environmental factors may include diet amount (calories, macronutrients), physical fitness abilities (e.g. stretching, flexibility, heart rate recovery), family type (adopted family or not, has siblings or not, lived with extended family during childhood), property and item ownership (has home or rents, has a smartphone or doesn't, has a car or doesn't).

Surveys also may be related to other environmental factors such as geographical, social-economic, or cultural factors. Geographical questions may include questions related to the birth location, family migration history, town, or city of users' current or past residence. Social-economic questions may be related to users' education level, income, occupations, self-identified demographic groups, etc. Questions related to culture may concern users' native language, language spoken at home, customs, dietary practices, etc.

For any survey questions asked, the computing server 130 may also ask an individual the same or similar questions regarding the traits and environmental factors of the ancestors, family members, other relatives, or friends of the individual. For example, a user may be asked about the native language of the user and the native languages of the user's parents and grandparents. A user may also be asked about the health history of his or her family members.

In addition to storing the survey data in the individual profile store 210, the computing server 130 may store some responses that correspond to data related to genealogical and genetics respectively to genealogical data store 200 and genetic data store 205.

The user profile data, survey response data, the genetic data, and the genealogical data may subject to the privacy and authorization setting from the users. For example, when presented with a survey question, a user may select to answer or skip the question. The computing server 130 may present users from time to time information regarding users' selection of the extent of information and data shared. The computing server 130 also may maintain and enforce one or more privacy settings for users in connection with the access of the user profile data, genetic data, and other sensitive data. For example, the user may pre-authorize the access of the data and may change the setting as wish. The privacy settings also may allow a user to specify (e.g., by opting out, by not opting in) whether the computing server 130 may receive, collect, log, or store particular data associated with the user for any purpose. A user may restrict her data at various levels. For example, in one level, the data may not be accessed by the computing server 130 for purposes other than displaying the data in the user's own profile. On another level, the user may authorize the anonymization of her data and participate in studies and researches conducted by the computing server 130 such as a large scale genetic study. In yet another level, the user may turn some portions of her genealogical data public to allow the user to be discovered by other users (e.g., potential relatives) and be connected in one or more family trees. Access or sharing of any information or data in the computing server 130 may also be subject to one or more similar privacy policies.

The sample pre-processing engine 215 receives and pre-processes data received from various sources to change the data into a format used by the computing server 130. For genealogical data, the sample pre-processing engine 215 may receive data from an individual via the user interface 115 of the client device 110. To collect the user data (e.g., genealogical and survey data), the computing server 130 may cause an interactive user interface on the client device 110 to display interface elements in which users can provide genealogical data and survey data. Additional data may be obtained from scans of public records. The data may be manually provided or automatically extracted via, for example, optical character recognition (OCR) performed on census records, town or government records, or any other item of printed or online material. Some records may be obtained by digitalizing written records such as older census records, birth certificates, death certificates, etc.

The sample pre-processing engine 215 may also receive raw data from genetic data extraction service server 125. The genetic data extraction service server 125 may perform laboratory analysis of biological samples of users and generate sequencing results in the form of digital data. The sample pre-processing engine 215 may receive the raw genetic datasets from the genetic data extraction service server 125. The human genome mutation rate is estimated to be 1.1*10^-8 per site per generation. This leads to a variant approximately every 300 base pairs. Most of the mutations that are passed down to descendants are related to single-nucleotide polymorphism (SNP). SNP is a substitution of a single nucleotide that occurs at a specific position in the genome. The sample pre-processing engine 215 may convert the raw base pair sequence into a sequence of genotypes of target SNP sites. Alternatively, the pre-processing of this conversion may be performed by the genetic data extraction service server 125. The sample pre-processing engine 215 identifies autosomal SNPs in an individual's genetic dataset. In some embodiments, the SNPs may be autosomal SNPs. In some embodiments, 700,000 SNPs may be identified in an individual's data and may be stored in genetic data store 205. Alternatively, in some embodiments, a genetic dataset may include at least 10,000 SNP sites. In another embodiment, a genetic dataset may include at least 100,000 SNP sites. In yet another embodiment, a genetic dataset may include at least 300,000 SNP sites. In yet another embodiment, a genetic dataset may include at least 1,000,000 SNP sites. The sample pre-processing engine 215 may also convert the nucleotides into bits. The identified SNPs, in bits or in other suitable formats, may be provided to the phasing engine 220 which phases the individual's diploid genotypes to generate a pair of haplotypes for each user.

The phasing engine 220 phases diploid genetic dataset into a pair of haploid genetic datasets and may perform imputation of SNP values at certain sites whose alleles are missing. An individual's haplotype may refer to a collection of alleles (e.g., a sequence of alleles) that are inherited from a parent.

Phasing may include a process of determining the assignment of alleles (particularly heterozygous alleles) to chromosomes. Owing to sequencing conditions and other constraints, a sequencing result often includes data regarding a pair of alleles at a given SNP locus of a pair of chromosomes but may not be able to distinguish which allele belongs to which specific chromosome. The phasing engine 220 uses a genotype phasing algorithm to assign one allele to a first chromosome and another allele to another chromosome. The genotype phasing algorithm may be developed based on an assumption of linkage disequilibrium (LD), which states that haplotype in the form of a sequence of alleles tends to cluster together. The phasing engine 220 is configured to generate phased sequences that are also commonly observed in many other samples. Put differently, haplotype sequences of different individuals tend to cluster together. A haplotype-cluster model may be generated to determine the probability distribution of a haplotype that includes a sequence of alleles. The haplotype-cluster model may be trained based on labeled data that includes known phased haplotypes from a trio (parents and a child). A trio is used as a training sample because the correct phasing of the child is almost certain by comparing the child's genotypes to the parent's genetic datasets. The haplotype-cluster model may be generated iteratively along with the phasing process with a large number of unphased genotype datasets. The haplotype-cluster model may also be used to impute one or more missing data.

By way of example, the phasing engine 220 may use a directed acyclic graph model such as a hidden Markov model (HMM) to perform phasing of a target genotype dataset. The directed acyclic graph may include multiple levels, each level having multiple nodes representing different possibilities of haplotype clusters. An emission probability of a node, which may represent the probability of having a particular haplotype cluster given an observation of the genotypes may be determined based on the probability distribution of the haplotype-cluster model. A transition probability from one node to another may be initially assigned to a non-zero value and be adjusted as the directed acyclic graph model and the haplotype-cluster model are trained. Various paths are possible in traversing different levels of the directed acyclic graph model. The phasing engine 220 determines a statistically likely path, such as the most probable path or a probable path that is at least more likely than 95% of other possible paths, based on the transition probabilities and the emission probabilities. A suitable dynamic programming algorithm such as the Viterbi algorithm may be used to determine the path. The determined path may represent the phasing result. U.S. Patent Application No. 15/519,099, entitled "Haplotype Phasing Models," filed on October 19, 2015, describes one possible embodiment of haplotype phasing.

The IBD estimation engine 225 estimates the amount of shared genetic segments between a pair of individuals based on phased genotype data (e.g., haplotype datasets) that are stored in the genetic data store 205. IBD segments may be segments identified in a pair of individuals that are putatively determined to be inherited from a common ancestor. The IBD estimation engine 225 retrieves a pair of haplotype datasets for each individual. The IBD estimation engine 225 may divide each haplotype dataset sequence into a plurality of windows. Each window may include a fixed number of SNP sites (e.g., about 100 SNP sites). The IBD estimation engine 225 identifies one or more seed windows in which the alleles at all SNP sites in at least one of the phased haplotypes between two individuals are identical. The IBD estimation engine 225 may expand the match from the seed windows to nearby windows until the matched windows reach the end of a chromosome or until a homozygous mismatch is found, which indicates the mismatch is not attributable to potential errors in phasing or in imputation. The IBD estimation engine 225 determines the total length of matched segments, which may also be referred to as IBD segments. The length may be measured in the genetic distance in the unit of centimorgans (cM). A unit of centimorgan may be a genetic length. For example, two genomic positions that are one cM apart may have a 1% chance during each meiosis of experiencing a recombination event between the two positions. The computing server 130 may save data regarding individual pairs who share a length of IBD segments exceeding a predetermined threshold (e.g., 6 cM), in a suitable data store such as in the genealogical data store 200. U.S. Patent Application No. 14/029,765, entitled "Identifying Ancestral Relationships Using a Continuous stream of Input," filed on September 17, 2013, and U.S. Patent Application No. 15/519,104, entitled "Reducing Error in Predicted Genetic Relationships," filed on April 13, 2017, describe example embodiments of IBD estimation.

Typically, individuals who are closely related share a relatively large number of IBD segments, and the IBD segments tend to have longer lengths (individually or in aggregate across one or more chromosomes). In contrast, individuals who are more distantly related share relatively fewer IBD segments, and these segments tend to be shorter (individually or in aggregate across one or more chromosomes). For example, while close family members often share upwards of 71 cM of IBD (e.g., third cousins), more distantly related individuals may share less than 12 cM of IBD. The extent of relatedness in terms of IBD segments between two individuals may be referred to as IBD affinity. For example, the IBD affinity may be measured in terms of the length of IBD segments shared between two individuals.

Community assignment engine 230 assigns individuals to one or more genetic communities based on the genetic data of the individuals. A genetic community may correspond to an ethnic origin or a group of people descended from a common ancestor. The granularity of genetic community classification may vary depending on embodiments and methods used in assigning communities. For example, in some embodiments, the communities may be African, Asian, European, etc. In another embodiment, the European community may be divided into Irish, German, Swedes, etc. In yet another embodiment, the Irish may be further divided into Irish in Ireland, Irish immigrated to America in 1800, Irish immigrated to America in 1900, etc. The community classification may also depend on whether a population is admixed or unadmixed. For an admixed population, the classification may further be divided based on different ethnic origins in a geographical region.

Community assignment engine 230 may assign individuals to one or more genetic communities based on their genetic datasets using machine learning models trained by unsupervised learning or supervised learning. In an unsupervised approach, the community assignment engine 230 may generate data representing a partially connected undirected graph. In this approach, the community assignment engine 230 represents individuals as nodes. Some nodes are connected by edges whose weights are based on IBD affinity between two individuals represented by the nodes. For example, if the total length of two individuals' shared IBD segments does not exceed a predetermined threshold, the nodes are not connected. The edges connecting two nodes are associated with weights that are measured based on the IBD affinities. The undirected graph may be referred to as an IBD network. The community assignment engine 230 uses clustering techniques such as modularity measurement (e.g., the Louvain method) to classify nodes into different clusters in the IBD network. Each cluster may represent a community. The community assignment engine 230 may also determine sub-communities. The computing server 130 saves the data representing the IBD network and clusters in the IBD network data store 235. U.S. Patent Application No. 15/168,011, entitled "Discovering Population Structure from Patterns of Identity-By-Descent," filed on May 28, 2016, describes one possible embodiment of community detection and assignment.

The community assignment engine 230 may also assign communities using supervised techniques. For example, genetic datasets of known genetic communities (e.g., individuals with confirmed ethnic origins) may be used as training sets that have labels of the genetic communities. Supervised machine learning classifiers, such as logistic regressions, support vector machines, random forest classifiers, and neural networks may be trained using the training set with labels. A trained classifier may distinguish binary or multiple classes. For example, a binary classifier may be trained for each community of interest to determine whether a target individual's genetic dataset belongs or does not belong to the community of interest. A multi-class classifier such as a neural network may also be trained to determine whether the target individual's genetic dataset most likely belongs to one of several possible genetic communities.

Reference panel sample store 240 stores reference panel samples for different genetic communities. A reference panel sample is a genetic data of an individual whose genetic data is the most representative of a genetic community. The genetic data of individuals with the typical alleles of a genetic community may serve as reference panel samples. For example, some alleles of genes may be over-represented (e.g., being highly common) in a genetic community. Some genetic datasets include alleles that are commonly present among members of the community. Reference panel samples may be used to train various machine learning models in classifying whether a target genetic dataset belongs to a community, in determining the ethnic composition of an individual, and in determining the accuracy in any genetic data analysis, such as by computing a posterior probability of a classification result from a classifier.

A reference panel sample may be identified in different ways. In some embodiments, an unsupervised approach in community detection may apply the clustering algorithm recursively for each identified cluster until the sub-communities contain a number of nodes that is smaller than a threshold (e.g., contains fewer than 1000 nodes). For example, the community assignment engine 230 may construct a full IBD network that includes a set of individuals represented by nodes and generate communities using clustering techniques. The community assignment engine 230 may randomly sample a subset of nodes to generate a sampled IBD network. The community assignment engine 230 may recursively apply clustering techniques to generate communities in the sampled IBD network. The sampling and clustering may be repeated for different randomly generated sampled IBD networks for various runs. Nodes that are consistently assigned to the same genetic community when sampled in various runs may be classified as a reference panel sample. The community assignment engine 230 may measure the consistency in terms of a predetermined threshold. For example, if a node is classified to the same community 95% (or another suitable threshold) of times whenever the node is sampled, the genetic dataset corresponding to the individual represented by the node may be regarded as a reference panel sample. Additionally, or alternatively, the community assignment engine 230 may select N most consistently assigned nodes as a reference panel for the community.

Other ways to generate reference panel samples are also possible. For example, the computing server 130 may collect a set of samples and gradually filter and refine the samples until high-quality reference panel samples are selected. For example, a candidate reference panel sample may be selected from an individual whose recent ancestors are born at a certain birthplace. The computing server 130 may also draw sequence data from the Human Genome Diversity Project (HGDP). Various candidates may be manually screened based on their family trees, relatives' birth location, other quality control. The principal component analysis may be used to creates clusters of genetic data of the candidates. Each cluster may represent an ethnicity. The predictions of the ethnicity of those candidates may be compared to the ethnicity information provided by the candidates to perform further screening.

The ethnicity estimation engine 245 estimates the ethnicity composition of a genetic dataset of a target individual. The genetic datasets used by the ethnicity estimation engine 245 may be genotype datasets or haplotype datasets. For example, the ethnicity estimation engine 245 estimates the ancestral origins (e.g., ethnicity) based on the individual's genotypes or haplotypes at the SNP sites. To take a simple example of three ancestral populations corresponding to African, European and Native American, an admixed user may have nonzero estimated ethnicity proportions for all three ancestral populations, with an estimate such as [0.05, 0.65, 0.30], indicating that the user's genome is 5% attributable to African ancestry, 65% attributable to European ancestry and 30% attributable to Native American ancestry. The ethnicity estimation engine 245 generates the ethnic composition estimate and stores the estimated ethnicities in a data store of computing server 130 with a pointer in association with a particular user.

In some embodiments, the ethnicity estimation engine 245 divides a target genetic dataset into a plurality of windows (e.g., about 1000 windows). Each window includes a small number of SNPs (e.g., 300 SNPs). The ethnicity estimation engine 245 may use a directed acyclic graph model to determine the ethnic composition of the target genetic dataset. The directed acyclic graph may represent a trellis of an inter-window hidden Markov model (HMM). The graph includes a sequence of a plurality of node groups. Each node group, representing a window, includes a plurality of nodes. The nodes representing different possibilities of labels of genetic communities (e.g., ethnicities) for the window. A node may be labeled with one or more ethnic labels. For example, a level includes a first node with a first label representing the likelihood that the window of SNP sites belongs to a first ethnicity and a second node with a second label representing the likelihood that the window of SNPs belongs to a second ethnicity. Each level includes multiple nodes so that there are many possible paths to traverses the directed acyclic graph.

The nodes and edges in the directed acyclic graph may be associated with different emission probabilities and transition probabilities. An emission probability associated with a node represents the likelihood that the window belongs to the ethnicity labeling the node given the observation of SNPs in the window. The ethnicity estimation engine 245 determines the emission probabilities by comparing SNPs in the window corresponding to the target genetic dataset to corresponding SNPs in the windows in various reference panel samples of different genetic communities stored in the reference panel sample store 240. The transition probability between two nodes represents the likelihood of transition from one node to another across two levels. The ethnicity estimation engine 245 determines a statistically likely path, such as the most probable path or a probable path that is at least more likely than 95% of other possible paths, based on the transition probabilities and the emission probabilities. A suitable dynamic programming algorithm such as the Viterbi algorithm or the forward-backward algorithm may be used to determine the path. After the path is determined, the ethnicity estimation engine 245 determines the ethnic composition of the target genetic dataset by determining the label compositions of the nodes that are included in the determined path. U.S. Patent Application No. 15/209,458, entitled "Local Genetic Ethnicity Determination System," filed on July 13, 2016, describes an example embodiment of ethnicity estimation.

The front-end interface 250 may display various results determined by the computing server 130. The results and data may include the IBD affinity between a user and another individual, the community assignment of the user, the ethnicity estimation of the user, phenotype prediction and evaluation, genealogical data search, family tree and pedigree, relative profile and other information. The front-end interface 250 may be a graphical user interface (GUI) that displays various information and graphical elements. The front-end interface 250 may take different forms. In one case, the front-end interface 250 may be a software application that can be displayed at an electronic device such as a computer or a smartphone. The software application may be developed by the entity controlling the computing server 130 and be downloaded and installed at the client device 110. In another case, the front-end interface 250 may take the form of a webpage interface of the computing server 130 that allows users to access their family tree and genetic analysis results through web browsers. In yet another case, the front-end interface 250 may provide an application program interface (API).

### EXAMPLE MATCH CLUSTERS GENERATION AND DETERMINATION

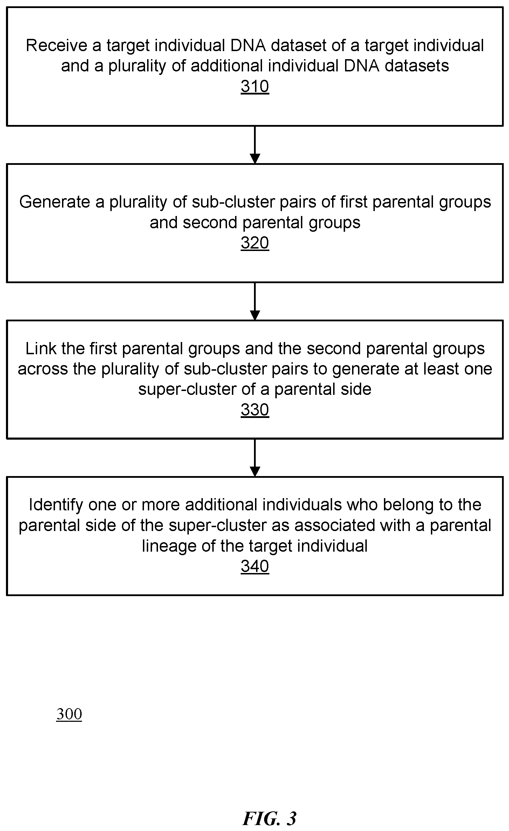

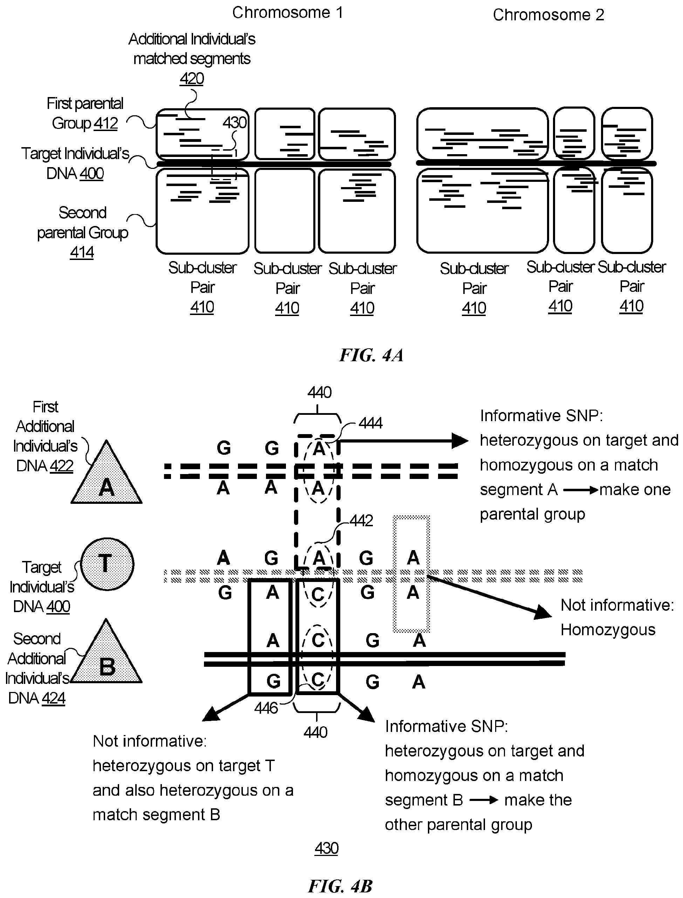

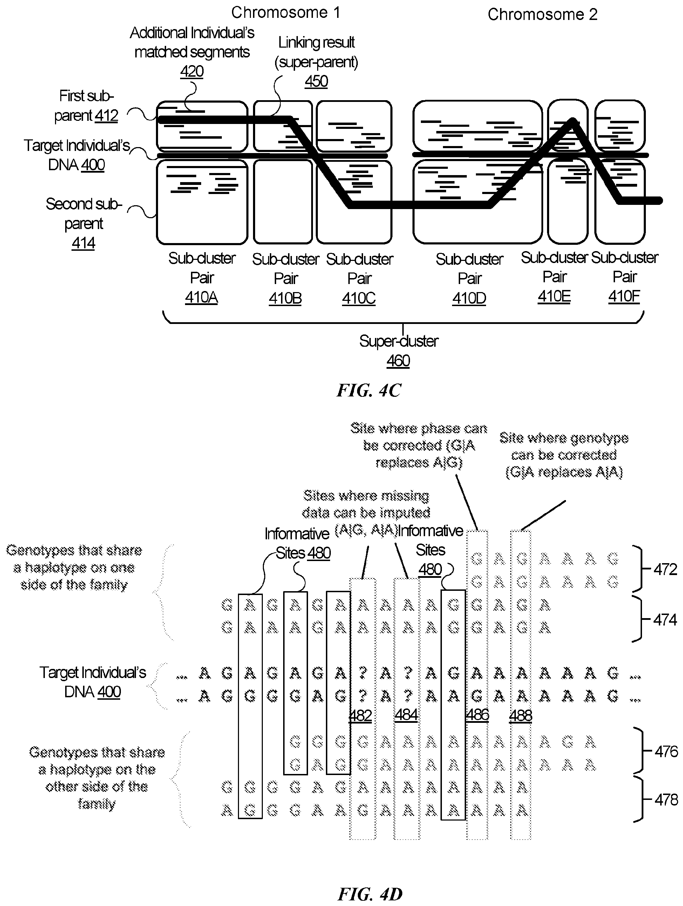

FIG. 3 is a flowchart depicting an example process 300 for generating match clusters for identifying parental lineages of ancestors or relatives correspond to a target individual, in accordance with some embodiments. FIGS. 4A, 4B, and 4C are conceptual diagrams comparing segments of DNA data between two or more potential relatives and a target individual. The flowchart in FIG. 3 will be discussed in conjunction with FIGS. 4A, 4B, and 4C.

In some embodiments, the computing server 130 may classify individuals who may be related to a target individual to a first parental side and a second parental side of the target individual by comparing the DNA datasets of the individuals and of the target individual. Based on the classification, the computing server 130 may identify which of the first parental side or the second parental side is the paternal side or the maternal side. The computing server 130 may add metadata to datasets corresponding to individuals to identify the connection between the individuals and the target individual. The computing server 130 may also provide notifications or graphical representations of the results indicating one or more individuals may be relatives of the user (the target individual). In some embodiments, the process described may classify potential relatives to one of the parental sides without the DNA dataset of either parent of the target individual. In other words, in some embodiments, the DNA datasets of other individuals may be directly compared to the DNA dataset of the target individual in classifying whether those individuals belong to a first or second parental side.

By way of example, the computing server 130 may receive 310 a target individual DNA dataset and additional individual DNA datasets, such as by retrieving the DNA datasets from a genetic data store 205. The target individual DNA dataset may include data of a plurality of allele sites of interests such as SNP sites of interest. Some of the allele sites may be homozygous while others may be heterozygous. The computing server 130 also may identify a number of additional individuals who may be related to the target individuals by identity by descent (IBD). The computing server 130 may receive a plurality of DNA datasets of those individuals (referred to as additional individual DNA datasets, in contrast to the target individual DNA dataset).

By way of example, the computing server 130 may retrieve a target genotype sequence in the DNA dataset of the target individual. The target genotype sequence may be biallelic. The computing server 130 may also retrieve a plurality of genotype sequences of the DNA datasets of additional individuals. Each site in various sequences may be homozygous for major alleles, heterozygous, or homozygous for the minor allele, and in some cases can be missing-not called by the lab, not otherwise imputed by the computing server 130. In some cases, the major allele is whichever is more common in a population. In other cases, the designation of major or minor can be arbitrary. Any genotype sequence may be referred to as a DNA dataset.

To classify an additional individual DNA dataset to a parental side of the target individual, the computing server 130 may perform a series of operations such as phasing, sequence matching, and clustering, linking. For instance, after receiving the target individual DNA dataset, the computing server 130 may divide the target individual DNA dataset into a plurality of genetic loci. For a genetic locus, the computing server 130 may scan through different additional individual DNA datasets to see if there are DNA datasets that have a matched segment. The computing server 130 may set a predetermined number as a threshold for considering whether a segment is a match. For example, in order to qualify as a match, a DNA dataset may need to include a sequence of alleles at multiple consecutive SNP sites that overlap with some portion of the target individual DNA dataset at the genetic locus.

The computing server 130 may classify more than one additional individual DNA dataset that has a matched segment that overlaps the target individual DNA dataset at the genetic locus as matches to the target individual. Those classified DNA datasets collectively may be referred to a sub-cluster pair because the computing server 130 may further classify those classified DNA datasets to a first parental group and a second parental group (e.g., a pair of a first group of DNA datasets classified to the first parental side and a second group of DNA datasets classified to the second parental side). A sub-cluster pair of parental groups may simply be referred to as a sub-cluster. A parental group in a sub-cluster may be referred to as a sub-parent group, or simply a sub-parent.

The computing server may generate 320 a plurality of sub-cluster pairs, each pair including a first parental group and a second parental group. FIG. 4A illustrates a conceptual diagram for multiple sub-cluster pairs 410. Thick horizontal lines in FIG. 4A represent the target individual's DNA 400. Thin and shorter horizontal lines represent additional individuals' matched segments 420. Horizontal lines 420 at the same vertical level represent different matched segments of the same additional individual. Each sub-cluster pair 410 may correspond to a segment of the target individual's DNA 400. The segment may correspond to one or more genetic loci. Each sub-cluster pair 410 has a first parental group 412 and a second parental group 414. For each segment that corresponds to a sub-cluster pair 410, the computing server 130 may identify additional individuals' matched segments 420 that match (e.g., matched by IBD) the target individual's DNA 400 and classify the matched segments to one of the two parental groups 412 or 414.

FIG. 4B illustrates an example process of classifying matched segments of additional individuals to one of the two parental groups 412 or 414, in accordance with some embodiments. FIG. 4B is a conceptual diagram illustrating an enlarged view of a region 430 in FIG. 4A, which includes the target individual's DNA 400, a first additional individual's DNA 422 (a first matched segment), and a second additional individual's DNA 424 (a second matched segment). In some embodiments, the computing server 130 may use one or more heterozygous allele sites of the target individual DNA dataset to classify different matched segments into two different parental groups 412 and 414. For example, the computing server 130 may identify a particular heterozygous allele site (e.g., 442) of the target individual DNA dataset at a genetic locus. The heterozygous allele site 442 includes a first allele (e.g., A) and a second allele (e.g., C) that is different from the first allele. The computing server 130 may assign the first allele as the first parental side and the second allele as the second parental side.

The computing server 130 may use an informative SNP site to for the classification of two parental sides. In some embodiments, to separate two parental sides, the computing server 130 may identify an allele site that has a heterozygous allele for the target individual and homozygous alleles at the same site of one or more matched individuals. Taking the third site 440 in FIG. 4B as an example, the computing server 130 may start with a heterozygous allele site 442 (A-C) of the target individual DNA. The computing server 130 identifies that a first matched segment of a first additional individual has homozygous (A-A) alleles 444 at the allele site 440 and classifies the matched dataset to the first parental side. Likewise, the computing server 130 identifies that a second matched segment of a second additional individual has homozygous (C-C) alleles 446 at the allele site 440 and classifies the second matched segment to the second parental side. In some embodiments, an informative SNP site may be a heterozygous allele site of the target individual DNA dataset that has at least two corresponding additional DNA datasets of two potential relatives who each has homozygous alleles at the site. While for the particular case shown in FIG. 4B that the homozygous alleles of the two matched individuals are different (e.g., one with A-A and another with C-C), in some cases, the homozygous alleles of the two matched individuals may be the same (e.g., both with A-A or both with C-C). If the computing server 130 identifies a second matched individual whose DNA dataset also has a homozygous allele at the target allele site but the allele is different from the first matched individual (e.g., the first matched individual is A-A and the second matched individual is C-C), then those two match individuals may correspond to two parental sides of the target individual. If the computing server 130 identifies a second matched individual whose DNA dataset also has a homozygous allele at the target allele site and the allele is the same as the first matched individual (e.g., both individuals have A-A), then those two match individuals may correspond to the same parental side of the target individual.

In classifying one or more candidate additional DNA datasets to either parental group of the target individual, the computing server 130 may break those one or more candidate additional DNA datasets into segments if matching fails (e.g., a candidate matched segment 420 fails to match the haplotype of the target individual) as the computing server 130 continues to examine the sequences. By way of example, the computing server 130 may retrieve a plurality of candidate additional DNA datasets of other individuals that are contiguous subsets of SNPs corresponding to the target individual's sequence 400. A candidate matched segment 420 (a sequence from DNA dataset of an additional individual) may share the same haplotype on the same parental side with the target genotype sequence for a length that exceeds a predetermined threshold. For example, the computing server 130 may begin at the informative heterozygous site A-C 442 of the target individual's sequence 400. The computing server 130 may classify candidate matched segments 420 that have A-A at the target site 440 and identify this group of candidate matched segments 420 as the first parental side. The computing server 130 may also classify other candidate matched segments 420 that have C-C at the target site 440 to the second parental side. At this point, in some cases, not all retrieved candidate matched segments 420 are grouped yet because some candidate matched segments 420 have heterozygous alleles at the target site 440 or have missing data at the target site 440. The computing server 130 may move along the target individual's sequence 400 to identify another heterozygous site (e.g., a site having alleles C-T, not shown in FIG. 4B). At this second heterozygous site, additional candidate matched segments 420 that were not classified at the first heterozygous site 440 (due to the candidate's site being heterozygous or due to missing data) may be classified. Also, the computing server 130 determines classified candidate matched segments 420 that are contradicting each other. For example, two candidate matched segments 420 may be classified to the same parental side due to both having A-A at the first site 440. Yet, at the site that corresponds to the second heterozygous site of the target individual, the two candidate matched segments 420 have contradicting homozygous alleles (e.g., one having C-C and another having T-T). In such a case, the computing server 130 breaks one of the two candidate matched segments 420 into segments that separate the conflicting sites. As a result, an additional individual's matched segments 420 at the same vertical level (i.e., representing the same individual) may be broken into various segments.

The contradiction in various sites among different candidate matched segments 420 may be attributable to various reasons. For example, the target or candidate sequences may be wrong due to genotyping error or imputation error. A candidate matched segment 420 may have incorrect endpoints (e.g., the sequence extends beyond where the haplotype sharing really stops). The candidate sequences may share the alleles with the target individual's sequence with both parents but the candidate matched segments 420 switch at some point because of a recombination event in the family history. The last case may occur relatively frequently among matches between the target individual and other descendants of the target's parents (e.g., her siblings, nephews, children, etc.). Hence, the computing server 130 may break up a candidate matched segment 420 by inserting breakpoints to create two matched segments. In some cases, after inserting breakpoints, small segments that are shorter than a predetermined threshold may be discarded.

Put differently, each segment of the target individual's DNA dataset may include a number of informative SNPs. In some cases, not all alleles on the same matched segment 420 have the same parental group. For example, the first 30 SNPs might belong to the first parental group, but the next 20 SNPs might belong to the second parental group. There could be a number of reasons for this phenomenon: (1) the matched segment is from a descendent of the target individual and therefore, the match could be on both sides of the family and (2) the matched segment might be extended erroneously due to the IBD matching process, which allows match extension until a homozygous mismatch happens. In the second case, the part of the match that is wrong may not belong to either parent. The issue may be resolved by breaking up the matches at positions. These positions are selected by considering the evidence presented other matched segments overlapping the target individual at the loci of question. After matches are broken into segments that are consistently on only one parental side, only segments with length over a certain threshold (e.g., 5cM) are kept for further clustering into pairs of parental groups.

In choosing to add breakpoints to segments, the computing server 130 may try to reduce or minimize the number of segments that are broken at places where the segment really shares a haplotype with the target individual. Given the choice between breaking many matches and breaking a few, the computing server 130 may choose to break a few. The computing server 130 may also consider the confidence that a matched segment shares a haplotype with the target individual, which is lower near the endpoints (beginning and end) of the segment because the matched segments are generally estimated in a way that allows them to be too long on either or both side.

In some embodiments, after the candidate matched segments 420 are broken, there are no more conflicts. That means any pair of matches will either have the exact same homozygous genotype or the exact opposite homozygous genotype (i.e., homozygous for different alleles than each other) at the informative sites. The process of detecting conflicts may classify matches into two parental groups 412 and 414. In some embodiments, the matches in the same parental group share the same alleles at the sites within a segment that is between two breakpoints. Two matches in the opposite parental groups 412 and 414 have opposite alleles (as reflected in the heterozygous alleles in the target individual) within the segment between the two breakpoints.

The two opposite groups may constitute a sub-cluster pair 410. The first group of the sub-cluster pair 410 may be referred to as a first parental group 412 because the classified matches share the same haplotype with the target individual. The computing server 130 may carry out the classification process simultaneously for the second allele of the heterozygous allele sites within two breakpoints of the target individual's segment to classify other matches to a second parental group 414. The classified matches in the second parental group 414 may share the same haplotype with the target individual but have the opposite haplotype of the first parental group 412. The first parental group 412 and the second parental group 414, both related to one or more heterozygous allele sites of the target individual, may be referred to as a pair of parental groups.

The group assignments in different sub-cluster pairs 410 do not always need to be unique. Each "side" (top vs. bottom in Fig. 4A) of a sub-cluster pair (sometimes these sides may be referred to "sub-groups" or "sub-parents") shares a haplotype inherited from one particular parent. However, in some embodiments, which haplotype belongs to father or mother may be undetermined at this point. For example, the top parental group 412 of the first sub-cluster 410 may belong to the father side while the top parental group 412 of the second sub-cluster 410 may belong to the mother side. When two sub-clusters 410 do not contain matches that overlap each other significantly, which sub-parent of a sub-cluster 410 corresponds to the same parent of the other sub-clusters may be difficult to determine. If two sub-clusters only overlap by a small amount (i.e., one or a few matches from either sub-clusters overlap with each other by a small number of SNPs), the matches may extend beyond the point where the two genotypes truly share a haplotype, so inference could be error-prone. As such, in some embodiments, a threshold may be set for defining a sub-cluster 410. For example, sub-clusters 410 may be a set of matched segments such that each overlaps another by a significant number of informative sites. The minimum number of overlap informative sites may correspond to a predetermined threshold (e.g., 40). The threshold may also be in the range of 5, 10, 20, 40, 50, 100, 150, 200, 500, 1000, etc. To build or expand one or more sub-clusters 410, the computing server 130 may start with each matched segment in its own sub-cluster and go through other matches. If the matches overlap by more than a threshold number of informative sites, the computing server 130 may join both of their entire sub-clusters into one.

The computing server 130 may further repeat the breaking of candidate matched segments 420, identification of matches, and building and expanding of sub-clusters for other genetic loci. As such, the computing server 130 may generate a plurality of pairs of parental groups across different genetic loci. Each pair may become a sub-cluster pair 410. For example, FIG. 4A illustrates a plurality of sub-cluster pairs 410. Each chromosome may be divided into a plurality of intervals. In the particular example shown in FIG. 4A, for illustration, each chromosome is divided into three intervals, but a chromosome may be divided into many more intervals. In some embodiments, the division may correspond to known genetic loci. In other embodiments, other ways to divide the chromosome are also suitable. In the particular embodiment shown in FIG. 4A, the computing server 130 generates six sub-cluster pairs 410 of parental groups for two chromosomes.

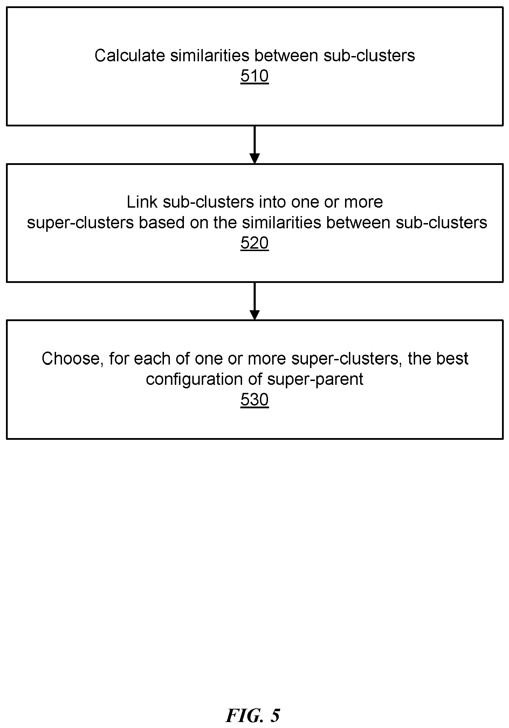

The computing server 130 links 330 the first parental groups 412 and the second parental groups 414 across multiple sub-cluster pairs 410 to generate at least one super-cluster of a parental side. In some embodiments, linking of the sub-clusters 410 may refer to classifying the parental groups in each sub-cluster 410 to one of the parental sides. For example, referring to FIG. 4C, while the computing server 130 classifies matched segments into one of the parental groups in each sub-cluster 410, without linking, the computing server 130 may not know if the top parental group 412 of the first sub-cluster pair 410A belong with the first parental side or the second parental side. The top parent group 412 of the second sub-cluster pair 410A, even though currently is placed on the north side of the parental side, may in fact belong to the south side of the parental side. There may be cases where the two lower parental groups may not belong to the same parental side. The reasons are that there are people who belong to the lower parental of the first sub-cluster 410A and also to the upper parental group of the another sub-cluster (e.g., third sub-cluster 410C) and/or there are many matches between the individuals belonging to the lower parental group of the first sub-cluster 410A and individuals belonging to the upper parental group of the third sub-cluster 410C. In some embodiments, the computing server 130 groups two or more sub-clusters 410 into a super-cluster based on any individuals who have multiple matched segments with the target individual in multiple sub-clusters 410 or based on matched segments among the relatives that may or may not be shared with the target individual. The linking of sub-clusters to super-cluster may be carried out using a heuristic scoring approach, a bipartite graph approach, or other suitable approaches that will be discussed in FIG. 5 through FIG. 10. The linkage process may be based on similarities among the parental groups across the plurality of sub-cluster pairs. The similarities may be based on a number of common additional DNA datasets classified in different parental groups across the plurality of pairs. An example of a linking result of the linkage of sub-clusters into a super-cluster 460 is shown in FIG. 4C as a thick line 450. The linked parental groups by the linking result 450 may also be referred to as a super-parent group or simply super-parent 450. Each parental group 412 or 414 in a sub-cluster pair 410 may be referred to as a sub-parent group. A super-cluster that includes a pair of parental sides may be referred to as a pair of super-parents. Individuals whose DNA datasets are classified to one of the parental sides of the super-cluster are likely individuals that are related to the target individual on the parental side. The computing server 130 may identify one or more individuals who belong to a parental side of the target individual. The identified individuals have DNA datasets that belong to the parental side of the super-cluster.

By way of example, in some embodiments, linking of the sub-clusters 410 into one or more super-parents may include randomly assigning the super-parent groups to each sub-cluster 410, then switch the super-parent group assignment of a sub-cluster that increases the sum of similarity between all sub-parent groups in the same super-parent groups by the most, and continue switching until no switch increases the sum of similarity any more. The process can be repeated over and over until with different random initial assignments in an attempt to find a better optimal super-parent group assignment for all sub-clusters. Sub-clusters that have sub-parent groups that have a nonzero similarity are linked together into the same super-cluster. There will be generally one or more super-clusters. Two different super-clusters remain disjoint because there is no nonzero similarity score linking any sub-parent group from one super-cluster to any sub-parent group of the other. This approach is discussed in further detail below as an example heuristic scoring method.

After one or more super-parent groups are identified, the computing server 130 may identify 340 one or more additional individuals who belong to the parental side of the super-cluster as associated with a parental lineage of the target individual. The computing server 130 may assign metadata to additional individuals' DNA datasets to associate the dataset with a parental side of the target individual. For example, the computing server 130 may assign metadata to one or more additional individual datasets. The metadata may specify that the one or more additional individual datasets are connected to the target individual dataset by the parental side of the super-cluster.

In some cases, after linking sub-clusters 410, there are individuals whose matched segments 420 might belong to two sides of the family. There are a number of reasons why these individuals have matched segments 420 belonging to both parental groups: (1) the individuals might be descendants of the target individuals such as nieces or nephews; (2) the parents of the target individual might share IBD. The second reason can lead to individuals matching with the target individual as well as both of the target individual's parents. The method identifies individuals whose matched segments 420 belong to both sides of the family by finding individuals who have segments in both super-parents. These individuals may be removed from their sub-clusters 410 and the process of linking sub-clusters 410 into super-cluster 460 is repeated.

The computing server 130 may further identify whether a parental side (e.g., a super-parent 450) is the paternal side or maternal side. One or more approaches may be used to enable such identification. In one embodiment, the computing server 130 access genealogical data of the target individual to identify at least one individual in the genealogical data who belong to the super-parent 450. Based on the genealogical data, the identified individual belongs to either a paternal side or maternal side of the target individual. In another embodiment, the computing server 130 may transmit, to the target individual (e.g., a user of the computing system) or another user, an inquiry about a relationship between the target individual and one of the identified additional individuals belonging to the super-parent. For example, the computing server 130 may ask a user whether one or more close relatives belong to the maternal side or the paternal side. In yet another embodiment, the computing server 130 may examine the genetic locus of sex chromosomes or mitochondrial DNA in the super-parent to determine the parental side. For example, if a parental side of a super-parent includes some segment of the Y-chromosome, the computing server 130 may designate the parental side as the paternal side. Likewise, if a parental side of a super-parent includes some segment of mitochondrial DNA, the computing server 130 may designate the parental side as the maternal side. In another embodiment, the computing server 130 may determine an ethnicity of one or more identified additional individuals belonging to the super-parent. The server may also ask the target individual or another user if the user knows her parents' or grandparents' genetic communities. This information may also be used to identify the maternal side or parental side because a super-parent 450 may be clustered or otherwise classified into one of the genetic communities using community assignment engine 230 or ethnicity estimation engine 245.

In one embodiment, in determining a parental side, the computing server 130 may rely on genealogical data such as pedigree and family tree information. The computing server 130 may collect the number of matched segments 420 that can be assigned to the maternal/paternal side by the genealogical data to determine which side of the family a sub-cluster belongs to. A machine learning model may be trained to a sub-cluster level classifier to assign top/bottom sub-cluster to maternal/paternal side with a probability given the number of maternal/paternal segments found in top/bottom sub-cluster. The prediction result is the assignment of the maternal/paternal side of the family for a top/bottom sub-cluster, which can be determined to use or not based on its classification probability. Similarly, a machine learning model (e.g. logistic regression) may be trained as a super-cluster-level classifier to assign top/bottom super-cluster to the maternal/paternal side of the family.

In determining whether a DNA dataset has a matched segment that matches the target individual DNA dataset, the computing server 130 may use a predetermined number of consecutive sites as a threshold to determine which parental group the match belongs to. In one embodiment, the predetermined number may be set as a fixed number such as 40 allele sites. In another embodiment, the computing server 130 may determine the threshold amount based on validation data. For example, the computing server 130 may examine different threshold amounts to generate different sub-clusters and super-clusters to determine an appropriate level of threshold that leads to the best accuracy in identifying individuals into different parental sides.

### EXAMPLE HAPLOTYPE PHASING AND GENOTYPE IMPUTATION

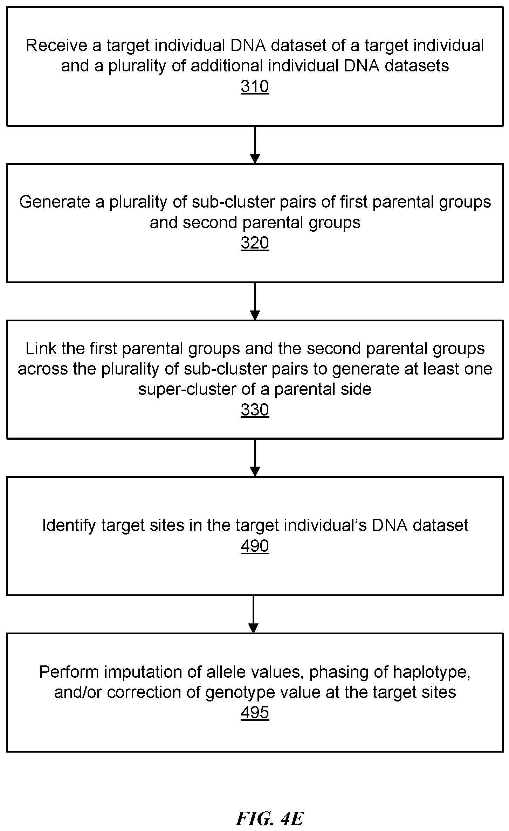

The process illustrated in FIG. 4B associated with assigning additional individuals' matched segments in one of two parental sides using informative sites 442 may also be used for haplotype phasing and imputation of allele values for the target individual. FIG. 4D is a conceptual diagram illustrating a process of haplotype phasing and imputation of missing values for the target individual, in accordance with some embodiments. FIG. 4E is a flowchart depicting an example haplotype phasing and genotype imputation process, in accordance with some embodiments. FIG. 4D is discussed in conjunction with FIG. 4E.

The computing server 130 may perform one or more steps described in the process 300, such as receiving 310 a target individual DNA dataset of a target individual and a plurality of additional individual DNA datasets, generating 320 at least one sub-cluster pair of first parental group and second parental group. In some embodiments, the computing server 130 may also link 330 the first parental groups and the second parental groups across the plurality of sub-cluster pairs to generate one or more super-clusters if the computing server 130 tries to determine a long-range haplotype. In some cases, if the desired haplotype range is shorter than the range of a sub-cluster, no inter-sub-cluster linking 330 is performed.

For example, in generating 320 at least a sub-cluster pair, the computing server 130 assigns matched segments to two parental groups. In FIG. 4D, the computing server 130 may identify heterozygous sites in the target individual's DNA 400. The computing server 130 identifies additional individuals' matched segments 472, 474, 476, and 478. Based on the heterozygous alleles of the target individual's DNA 400, the computing server 130 classifies the matched segments to one of the two sides of the family (e.g., one of the two parental groups). The computing server 130 may identify one or more informative sites 480 in which the target individual's DNA at those sites are heterozygous while the matched segments 472, 474, 476, or 478 at those sites are homozygous. In some cases, the computing server 130 may also identify homozygous sites of the target individual that match the additional individuals.

The computing server 130 may identify 490 target sites in the target individual's DNA dataset. By way of non-limiting example, the computing server 130 may select target sites based on a distance between a candidate site and another site that the computing server 130 deems as a high-confidence site. High-confidence sites may be informative sites 480 or homozygous sites in which both the target individual and the additional individuals have the same allele. Target sites are in the same proximity of the high-confidence sites, such as sites that are within a threshold distance from at least one information site 480 or sites that are belong to the same sub-cluster or the same genetic loci. The computing server 130 may perform 495 imputation of allele values, phasing of haplotype, and/or correction of genotype value at the target sites. For example, at the target individual's sites 482 and 484, the sequencing result does not provide a genotype value at those sites. Based on the matched segments 472, 474, 476, and/or 478 that are assigned to two different sides of the family, the computing server 130 imputes that the haplotype values at the first missing site 482 as A|G by identifying homozygous matched segments at those sites. Likewise, the computing server 130 imputes the haplotype values at the second missing site 484 as A|A. The computing server 130 may also phase or correct phasing error performed by phasing engine 220 using the matched segments in the sub-cluster. For example, at the heterozygous site 486, the values A|G can be either unphased or phased with an error. The computing server 130 reviews the homozygous allele values of matched segments 472, 474, 476, and/or 478 at the site 486. The computing server 130 determines that the correct phasing should be G|A instead of A|G. The computing server 130 may also use the matched segments to correct a genotyping error. For example, at the site 488, the genotyping result produced by sequencing is A|A. However, the matched segments 472 and 474 suggest that the alleles should be G|A.

In any cases when genotyping or haplotype phasing errors are detected, the computing server 130 may choose to override the genotype in the original data, choose to override the genotype in the phased data (often the diploid data have missing calls and the phased data do not), or choose to override the genotype in both the original and phased data. The computing server 130 may determine the extent of overriding data based on one or more factors. For example, the factors may include the number of matched segments support the identification of error, the number of matched segments on either side of the family, the number of matched segments being homozygous at the site where an error is found, and which alleles the matched segments are homozygous. The factors may also include whether the computing server 130 is changing a genotype assignment or not and what the original genotype is. The factors may further include the confidence in the IBD segments (e.g., how certain the computing server 130 is that the segment shares a haplotype with the target individual). The confidence in the IBD segments may be based on genotype data and supporting information, including but not limited to the proximity of the SNP in question to either end of the segment, the length of the segment, and the estimated amount of DNA shared with the same individual as the IBD segment in other places on the genome.

The use of match clustering and sub-cluster techniques for haplotype phasing described in FIG. 3 through FIG. 4B can improve the phasing method used by the phasing engine 220 by at least 35%. The match-clustering based haplotype phasing can also improve the performance of genetic communities and ethnicities used in community assignment engine 230 and ethnicity estimation engine 245.

### EXAMPLE HEURISTIC SCORING APPROACH

In some embodiments, the computing server 130 generates one or more super-clusters and their linking using a heuristic scoring method. FIG. 5 is a flowchart depicting an example process for linking sub-clusters to generate super-clusters, in accordance with some embodiments. The computing server 130 may calculate 510 the similarity between sub-parents of different sub-clusters 410. Each matched segment 420 in a sub-cluster 410 corresponds to a different relative of the target individual. In some embodiments, the similarity between sub-parents of two sub-clusters 410 may be based on a number of matched segments 420 whose corresponding relatives are shared between the two sub-parents in two sub-clusters 410. In other words, it is based on the number of matched segments in the two sub-cluster-sub-parents whose corresponding relatives are the same. For example, if a person has 2 segments in the upper parental group of sub-cluster 1 (***sub _1_p0***) and 5 segments in the lower parental group of sub-cluster 3 (***sub_3_p1***)*,* then the similarity score between these two sub-cluster-sub-parents groups (i.e. ***sub_1_p0*** and ***sub**_**3**_**p1***) is based on the 7 segments corresponding to this person. In some embodiments, the similarity between two sub-parents in sub-clusters 410 may be based on the number of matched segments 420 in one sub-parent whose corresponding relative is "a match" of the corresponding relative of a matched segment in the other sub-parents. In some embodiment, the similarity score is based on the number of matched segments in the two sub-parents whose corresponding relatives are matches of each other. For example, if a person *A* has segments in ***sub_1_p0*** and a person *B* has segments in ***sub_3**_**p1**,* and persons *A* and *B* match each other on X segments, then the similarity score between these two sub-cluster-sub-parents groups (i.e. ***sub_1_p0*** and ***sub_3_p1***) is based on the X segments shared between person A and person B. In some embodiments, the similarity between two sub-parents may be based on a combination of the number of matched segments in the two sub-parents whose corresponding relatives are the same, and the number of matched segments in the two sub-parents whose corresponding relatives are matches of each other. In some embodiments, the result of step 510 may be a similarity matrix Sim(i, j, k) where i, j are indices of sub-clusters. k = True if we compare sub-cluster i, top to sub-cluster j, top and sub-cluster i bottom to sub-cluster j bottom. k=False if we compare sub-cluster i, bottom to sub-cluster j, top and sub-cluster i top to sub-cluster j bottom.

The computing server 130 may link 520 sub-clusters 410 into one or more super-clusters 460 based on the similarities between sub-clusters 410. As a result of this step a sub-cluster 410 is assigned to a super-cluster 460 and a target individual can have one or more super-clusters 460 depending on the similarity between their sub-clusters 410.

The computing server 130 may choose 530 the best configuration (or one of the best) of super-cluster-super-parent for each of one or more super-clusters. A configuration of super-cluster-super-parent for a super-cluster represents a set of assignments of super-parents (e.g. first super-parent/second super-parent, 0/1, mother/father, true/false or any other appropriate values) to each of the sub-cluster-sub-parent groups within the super-cluster. The configuration indicates which sub-cluster-sub-parents correspond to a first super-parent and which ones correspond to a second super-parent. A sub-cluster 410 includes two sub-parent groups 412 and 414 (FIG. 4C), e.g. sub-cluster-sub-parent 0 and sub-cluster-sub-parent 1, wherein sub-cluster-sub-parent 0 is the part whose sub-parent is assigned (e.g., a first sub-parent), and wherein sub-cluster-sub-parent 1 is the part whose sub-parent is assigned (e.g., a second sub-parent). A super-cluster 460 may include two super-parent groups, such as super-parent 0 and super-parent 1, wherein super-parent 0 and super-parent 1 are the result of linking sub-clusters 410. A super-cluster 460 includes one or more sub-clusters 410. A super-parent 450 includes one or more sub-parents 412 or 414 that are linked. The similarity score of a super-cluster 460 is defined based on the sum of similarity scores between sub-clusters within each super-parent of the super-cluster 460.

In step 530, to find the best configuration of the linking of a super-cluster 460, the computing server 130 chooses a group of candidate configurations of super-parents. The computing server 130 selects the candidate configuration from the group of candidate configurations which results in the highest similarity score of the super-cluster as the best configuration of super-cluster-super-parent.

To find a candidate configuration for a super-cluster 460, the computing server 130 randomly assigns a super-parent (e.g. first super-parent or second super-parent) to each sub-parent 412 or 412 in various sub-clusters 410. The computing server 130 switches the assignment of the super-parent label if switching increases the similarity score of the super-cluster 460, wherein all possible switching of super-parents are iterated through. The configuration corresponding to the highest similarity score of the super-cluster is chosen as the candidate configuration. Switching an assignment of the super-parent to a sub-parent means that if for a sub-cluster ***sub_i*** with two sub-parents ***sub_i_p0*** and ***sub_i_p1**,* if initially a first super-parent is assigned to ***sub_i_p0*** and a second super-parent is assigned to ***sub_i_p1**,* after switching the assignment, the second super-parent is assigned to ***sub_i_p0*** and the first super-parent is assigned t*o **sub_i_p1.*** Similarly, if initially a first super-parent is assigned to ***sub_i_p1*** and a second super-parent is assigned t***o sub_i_p0**,* after switching the assignment, the second super-parent is assigned to ***sub_i_p1*** and the first super-parent is assigned t*o **sub_i_p0**.* Finding candidate configurations may be repeated for a predetermined number of times N (e.g. 1000 times) to have a group of candidate configurations. As a result of step 530, each segment has a super-cluster and super-parent assignment. This step may be repeated multiple times: starting with a random configuration, switching the assignment until the best configuration is achieved. The best resulting configuration among the multiple random restarts is selected as the final super-cluster and super-parent assignment.

### EXAMPLE BIPARTITE GRAPH APPROACH

In some embodiments, the computing server 130 generates one or more super-clusters and their linking using a bipartite graph approach. Two sub-parent combinations in two different sub-clusters 410 can either be on the same parental side of a target individual or they are on two different parental sides of the target individual. There are cases where a person can have segments in a sub-parent that match both sides of the family (e.g., niece and nephew). In some embodiments, the computing server 130 may assume that a sub-parent can only be on one side of the target individual's family. Consider each sub-parent combination as a node in a graph and the computing server 130 may connect all pairs of nodes that are on two different parental sides of the family. The problem of assigning each sub-parent to one side of a family becomes a problem of coloring the nodes of the graph with two colors such that any two nodes connected by an edge are colored differently. In some embodiments, the computing server 130 represents a graph that could be colored with only two colors as a bipartite graph. The computing server 130 constructs a graph with sub-cluster-sub-parents combinations as nodes such that the graph is bipartite.

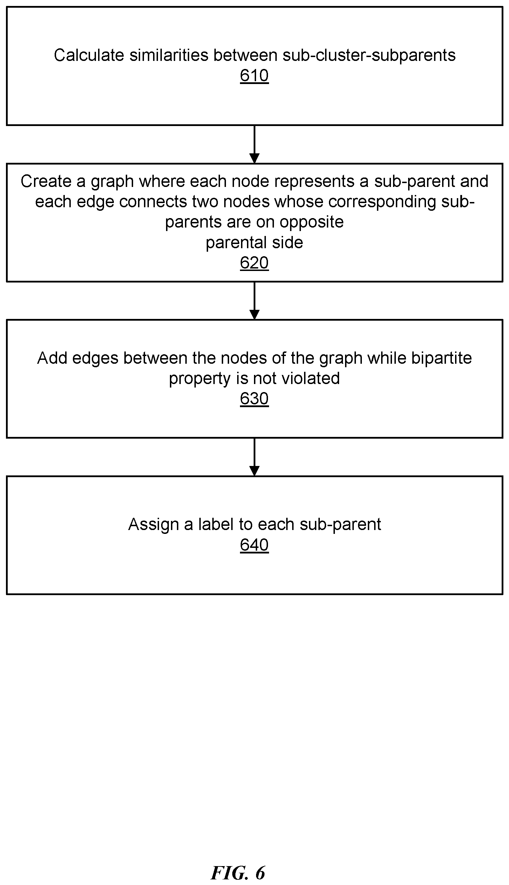

FIG. 6 is an example flowchart depicting a process for generating one or more super-clusters and their linking result using a bipartite graph by applying forward formulation, in accordance with some embodiments. The computing server may calculate 610 the similarities between all pairs of sub-parent combinations across two sub-clusters. Different embodiments may use various ways to calculate the similarity between sub-parents. In some embodiments, the similarity between two sub-parents may be based on a number of matched segments 420 whose corresponding relatives are shared between the two sub-parents. In other words, it is based on the number of matched segments in the two sub-parents whose corresponding relatives are the same. In some embodiments, the similarity between two sub-parents may be based on the number of matched segments 420 in one sub-parent whose corresponding relative is a match of the corresponding relative of a matched segment in the other sub-parents. In other words, it is based on the number of matched segments in the two sub-parents whose corresponding relatives are matches of each other. In some embodiments, the similarity between two sub-parents may be based on a combination of the number of matched segments in the two sub-parents whose corresponding relatives are the same, and the number of matched segments in the two sub-parents whose corresponding relatives are matches of each other.

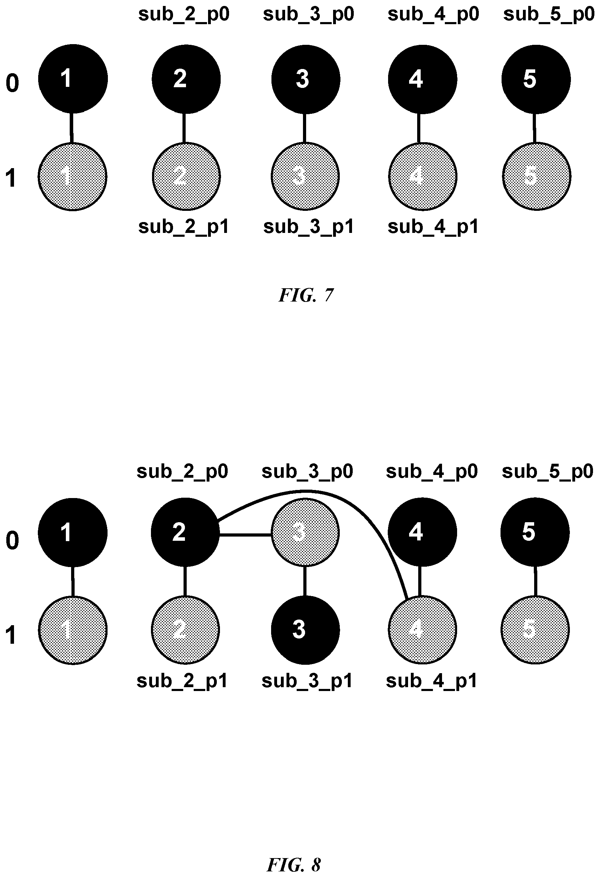

The computing server 130 may create 620 an initial graph where each node represents a sub-parent and each edge connects two nodes whose corresponding sub-parents are on the opposite parental side. Each node has a label (e.g. color, 1/0, any suitable binary labels) which represents a parental side (paternal side or maternal side). FIG. 7 is a conceptual diagram of an example initial bipartite graph using colors (black and grey) as the parental side label. The initial graph comprises nodes for sub-parents. For each sub-cluster ***sub_i*** with two sub-parents ***sub_i_p0*** and ***sub_i_p1, sub_i_p0*** and ***sub_i_p1*** are on the opposite sides of the family. Thus, the initial graph comprises edges between nodes corresponding to such sub-parents (e.g. ***sub_i_p0*** and ***sub_i_p1***) that are part of the same sub-cluster (e.g. ***sub_i)**.*

The computing server 130 may add 630 edges between nodes of the initial graph based on the similarity between sub-parents in different sub-clusters 410 until the bipartite property is violated. The computing server 130 iterates through a list of pairs of nodes from the highest to lowest similarity for their corresponding sub-parents. The computing server 130 adds edges between pairs of nodes while bipartite property is not violated in the graph. FIG. 8 shows an example of adding additional edges. The computing server 130 may start with the pairs of nodes with high similarity (the highest similarity). For example, if there is a high similarity between ***sub_2_p0*** and ***sub_4_p0**,* then ***sub_2_p0*** and ***sub_4_p1*** are on the opposite side of the family and an edge will be added between ***sub_2_p0*** and ***sub_4_p1**.* The computing server 130 may go down the list of the pairs of nodes from the highest to lowest similarity and continue to assign edges. If there is a high similarity between ***sub_2_p0*** and ***sub_3_p1**,* then ***sub_2_p0*** and ***sub_3**_**p0*** are on the opposite side of the family and an edge will be added between ***sub_2_p0*** and ***sub_3_p0**.* If the graph becomes non-bipartite (e.g., having an odd cycle), the computing server 130 may disconnect the most recently connected pairs.

Once all possible edges are added, the computing server 130 has completed a bipartite graph. The computing server 130 may assign 640 a parental-side label (e.g. color) to each sub-parent. Each label corresponds to a side of the family.

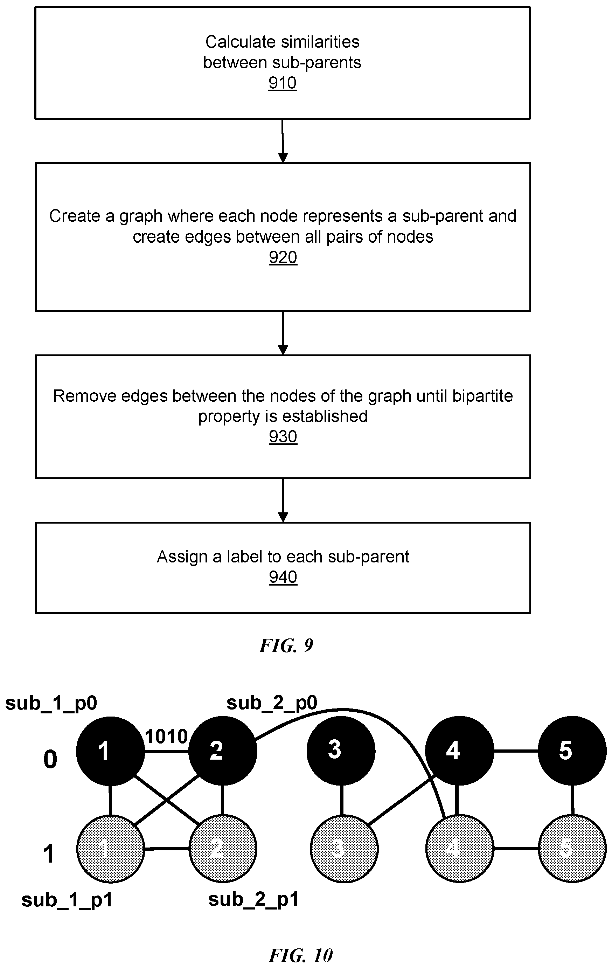

In some embodiments, the computing server 130 generates two or more super-clusters using a bipartite graph applying backward formulation. FIG. 9 is an example flowchart depicting a process for generating one or more super-clusters and their linking result using a bipartite graph by applying backward formulation, in accordance with some embodiments. The computing server 130 may calculate 910 the similarities between all pairs of sub-parent combinations. Different embodiments may use various ways to calculate the similarity between sub-parents. Step 910 may use various embodiments described in step 610 to calculate the similarity between sub-parents.

The computing server 130 may create 920 an initial graph where each node represents a sub-parent. Edges are created between all pairs of nodes in the initial graph to represent the potential sub-parents that are on the opposite parental sides.

The computing server 130 may remove 930 edges between nodes of the initial graph based on the similarity between sub-parents corresponding to the nodes. The computing server 130 may iterate through a list of pairs of nodes from highest to lowest similarity for their corresponding sub-parents. The computing server 130 may remove edges between the pair of nodes until bipartite property is established in the graph. FIG. 10 shows an example of removing an edge through the iteration. At an instance during iteration through the list of pairs of nodes from highest to lowest similarity for their corresponding sub-cluster-sub-parents, the computing server 130 reaches the pair of nodes ***sub_1_p0*** and ***sub_2_p0,*** as the one with the highest similarity. The computing server 130 then removes the edge 1010 between ***sub_1_p0*** and ***sub_2_p0*** because it violates bipartite property. In other words, because ***sub_1**_**p0*** and ***sub**_**2**_**p0*** are highly similar, their corresponding sub-cluster-sub-parents should be on the same side rather than the different side of the family, hence the edge 1010 is removed.

Once all possible edges that cause violation of bipartite property in the graph are removed, the computing server 130 has completed a bipartite graph, in which each sub-cluster-sub-parent combination is assigned a parental-side label (e.g. color) in step 940. Each label corresponds to a side of the family.

In some embodiments, the computing server 130 may generate two or more super-clusters using a combination of heuristic scoring and a bipartite graph. The computing server 130 runs the heuristic scoring method described above and calculates the similarity score of the resulting super-clusters. The computing server 130 also runs bipartite graph methods (forward formulation and/or backward formulation) and calculates the similarity score of the resulting super-clusters. The computing server 130 compares the calculated similarity scores and outputs the results corresponding to the best similarity score.

### COMPUTING MACHINE ARCHITECTURE

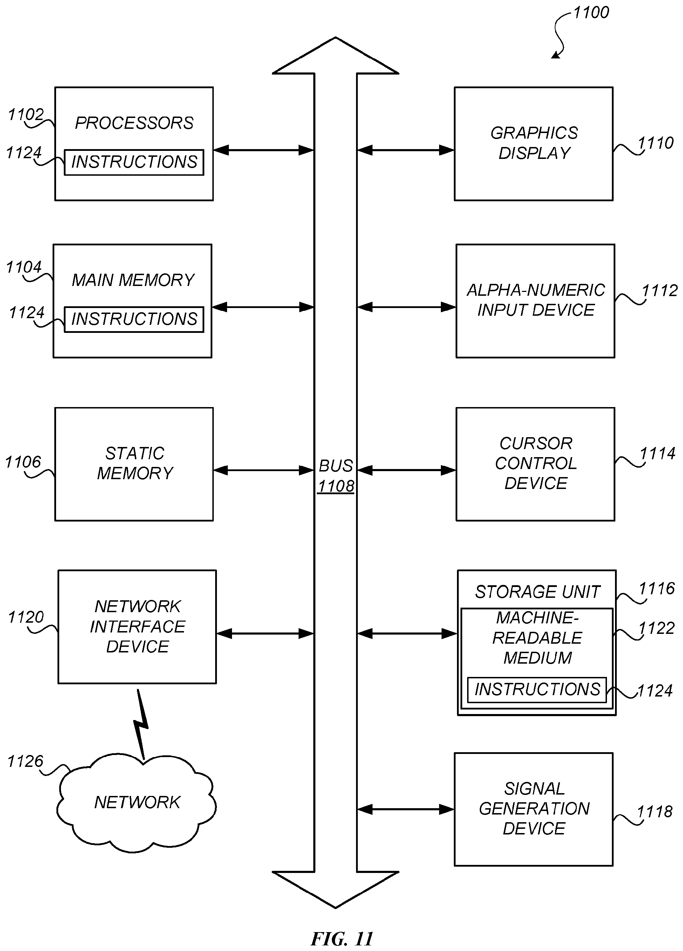

FIG. 11 is a block diagram illustrating components of an example computing machine that is capable of reading instructions from a computer-readable medium and execute them in a processor (or controller). A computer described herein may include a single computing machine shown in FIG. 11, a virtual machine, a distributed computing system that includes multiples nodes of computing machines shown in FIG. 11, or any other suitable arrangement of computing devices.

By way of example, FIG. 11 shows a diagrammatic representation of a computing machine in the example form of a computer system 1100 within which instructions 1124 (e.g., software, source code, program code, expanded code, object code, assembly code, or machine code), which may be stored in a computer-readable medium for causing the machine to perform any one or more of the processes discussed herein may be executed. In some embodiments, the computing machine operates as a standalone device or may be connected (e.g., networked) to other machines. In a networked deployment, the machine may operate in the capacity of a server machine or a client machine in a server-client network environment, or as a peer machine in a peer-to-peer (or distributed) network environment.

The structure of a computing machine described in FIG. 11 may correspond to any software, hardware, or combined components shown in FIGS. 1 and 2, including but not limited to, the client device 110, the computing server 130, and various engines, interfaces, terminals, and machines shown in FIG. 2. While FIG. 11 shows various hardware and software elements, each of the components described in FIGS. 1 and 2 may include additional or fewer elements.

By way of example, a computing machine may be a personal computer (PC), a tablet PC, a set-top box (STB), a personal digital assistant (PDA), a cellular telephone, a smartphone, a web appliance, a network router, an internet of things (IoT) device, a switch or bridge, or any machine capable of executing instructions 1124 that specify actions to be taken by that machine. Further, while only a single machine is illustrated, the term "machine" and "computer" may also be taken to include any collection of machines that individually or jointly execute instructions 1124 to perform any one or more of the methodologies discussed herein.

The example computer system 1100 includes one or more processors 1102 such as a CPU (central processing unit), a GPU (graphics processing unit), a TPU (tensor processing unit), a DSP (digital signal processor), a system on a chip (SOC), a controller, a state equipment, an application-specific integrated circuit (ASIC), a field-programmable gate array (FPGA), or any combination of these. Parts of the computing system 1100 may also include a memory 1104 that store computer code including instructions 1124 that may cause the processors 1102 to perform certain actions when the instructions are executed, directly or indirectly by the processors 1102. Instructions can be any directions, commands, or orders that may be stored in different forms, such as equipment-readable instructions, programming instructions including source code, and other communication signals and orders. Instructions may be used in a general sense and are not limited to machine-readable codes. One or more steps in various processes described may be performed by passing through instructions to one or more multiply-accumulate (MAC) units of the processors.

One and more methods described herein improve the operation speed of the processors 1102 and reduces the space required for the memory 1104. For example, the data processing techniques, algorithmic techniques and machine learning techniques described herein reduce the complexity of the computation of the processors 1102 by applying one or more novel techniques that simplify the steps in training, reaching convergence, and generating results of the processors 1102. The algorithms described herein also reduces the size of the models and datasets to reduce the storage space requirement for memory 1104.

The performance of certain of the operations may be distributed among the more than processors, not only residing within a single machine, but deployed across a number of machines. In some example embodiments, the one or more processors or processor-implemented modules may be located in a single geographic location (e.g., within a home environment, an office environment, or a server farm). In other example embodiments, the one or more processors or processor-implemented modules may be distributed across a number of geographic locations. Even though in the specification or the claims may refer some processes to be performed by a processor, this should be construed to include a joint operation of multiple distributed processors.

The computer system 1100 may include a main memory 1104, and a static memory 1106, which are configured to communicate with each other via a bus 1108. The computer system 1100 may further include a graphics display unit 1110 *(e.g.,* a plasma display panel (PDP), a liquid crystal display (LCD), a projector, or a cathode ray tube (CRT)). The graphics display unit 1110, controlled by the processors 1102, displays a graphical user interface (GUI) to display one or more results and data generated by the processes described herein. The computer system 1100 may also include alphanumeric input device 1112 (*e.g.,* a keyboard), a cursor control device 1114 (*e.g.,* a mouse, a trackball, a joystick, a motion sensor, or other pointing instrument), a storage unit 1116 (a hard drive, a solid state drive, a hybrid drive, a memory disk, etc.), a signal generation device 1118 (*e.g.,* a speaker), and a network interface device 1120, which also are configured to communicate via the bus 1108.

The storage unit 1116 includes a computer-readable medium 1122 on which is stored instructions 1124 embodying any one or more of the methodologies or functions described herein. The instructions 1124 may also reside, completely or at least partially, within the main memory 1104 or within the processor 1102 (*e.g.*, within a processor's cache memory) during execution thereof by the computer system 1100, the main memory 1104 and the processor 1102 also constituting computer-readable media. The instructions 1124 may be transmitted or received over a network 1126 via the network interface device 1120.

While computer-readable medium 1122 is shown in an example embodiment to be a single medium, the term "computer-readable medium" should be taken to include a single medium or multiple media (*e.g.,* a centralized or distributed database, or associated caches and servers) able to store instructions (*e.g*., instructions 1124). The computer-readable medium may include any medium that is capable of storing instructions (*e.g*., instructions 1124) for execution by the processors (*e.g*., processors 1102) and that cause the processors to perform any one or more of the methodologies disclosed herein. The computer-readable medium may include, but not be limited to, data repositories in the form of solid-state memories, optical media, and magnetic media. The computer-readable medium does not include a transitory medium such as a propagating signal or a carrier wave.

### ADDITIONAL CONSIDERATIONS

The foregoing description of the embodiments has been presented for the purpose of illustration; it is not intended to be exhaustive or to limit the patent rights to the precise forms disclosed. Persons skilled in the relevant art can appreciate that many modifications and variations are possible in light of the above disclosure.

Some portions of this description describe the embodiments in terms of algorithms and symbolic representations of operations on information. These operations and algorithmic descriptions, while described functionally, computationally, or logically, are understood to be implemented by computer programs or equivalent electrical circuits, microcode, or the like. Furthermore, it has also proven convenient at times, to refer to these arrangements of operations as engines, without loss of generality. The described operations and their associated engines may be embodied in software, firmware, hardware, or any combinations thereof.

Any of the steps, operations, or processes described herein may be performed or implemented with one or more hardware or software engines, alone or in combination with other devices. In some embodiments, a software engine is implemented with a computer program product comprising a computer-readable medium containing computer program code, which can be executed by a computer processor for performing any or all of the steps, operations, or processes described. The term "steps" does not mandate or imply a particular order. For example, while this disclosure may describe a process that includes multiple steps sequentially with arrows present in a flowchart, the steps in the process do not need to be performed by the specific order claimed or described in the disclosure. Some steps may be performed before others even though the other steps are claimed or described first in this disclosure. Likewise, any use of (i), (ii), (iii), etc., or (a), (b), (c), etc. in the specification or in the claims, unless specified, is used to better enumerate items or steps and also does not mandate a particular order.

Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components. These and other variations, modifications, additions, and improvements fall within the scope of the subject matter herein. In addition, the term "each" used in the specification and claims does not imply that every or all elements in a group need to fit the description associated with the term "each." For example, "each member is associated with element A" does not imply that all members are associated with an element A. Instead, the term "each" only implies that a member (of some of the members), in a singular form, is associated with an element A. In claims, the use of a singular form of a noun may imply at least one element even though a plural form is not used.

Finally, the language used in the specification has been principally selected for readability and instructional purposes, and it may not have been selected to delineate or circumscribe the patent rights. It is therefore intended that the scope of the patent rights be limited not by this detailed description, but rather by any claims that issue on an application based hereon. Accordingly, the disclosure of the embodiments is intended to be illustrative, but not limiting, of the scope of the patent rights.

## Claims

1. A computer-implemented method comprising:
receiving a target individual genotype dataset of a target individual;
identifying a plurality of identity-by-descent (IBD) matched segments (420), wherein each matched segment is shared between the target individual and an additional individual by identity-by-descent (IBD), wherein identifying the plurality of matched segments comprises comparing a plurality of additional individual genotype datasets with the target individual genotype dataset;
identifying the matched segments for the additional individuals who share a length of IBD segments with the target individual;
classifying the identified matched segments into a plurality of sub-cluster pairs (410), wherein each sub-cluster pair represents a genetic locus in a plurality of genetic loci, and wherein each sub-cluster pair has a first parental group comprising a first set of matched segments and a second parental group comprising a second set of matched segments;
generating a super-cluster (460) of a parental side by linking one or more first parental groups and one or more second parental groups across the plurality of sub-cluster pairs, wherein generating the super-cluster of the parental side comprises:
defining a candidate configuration for the super-cluster, the candidate configuration comprises candidate parental side assignments of parental groups across the plurality of sub-cluster pairs,
determining a number of common additional individual genotype datasets that are classified in the candidate configuration,
switching the candidate parental side assignments to increase number of common additional individual genotype datasets that are classified in the candidate configuration; and
generating a haplotype phasing of the target individual from the super-cluster of the parental side.

2. The computer-implemented method of claim 1, wherein classifying the identified matched segments into a plurality of sub-cluster pairs comprises:
identifying a heterozygous allele site in the target individual genotype dataset, the target individual genotype dataset having a first allele and a second allele at the heterozygous allele site,
classifying the matched segments having a first corresponding site that has the first allele and is homozygous to the first parental group, and
classifying the matched segments having a second corresponding site that has the second allele and is homozygous to the second parental group.

3. The computer-implemented method of claim 1 or claim 2, further comprising:
identifying the parental side as either paternal or maternal by one or more of the following:
accessing genealogical data of the target individual to identify at least one individual in the genealogical data who belong to the parental, the at least one identified individual belonging to either a paternal side or maternal side of the target individual according to the genealogical data,
transmitting, to a user, an inquiry about a relationship between the target individual and one of the identified additional individuals belonging to the parental side,
examining a genetic locus of sex chromosomes in the parental side to determine whether the parental side is paternal or maternal,
examining a genetic locus of mitochondrial DNA in the parental side to determine whether the parental side is paternal or maternal,
determining an ethnicity of one or more identified additional individuals belonging to the parental side, and/or
transmitting, to a user, an inquiry about a genetic community to which the target individual belongs.

4. The computer-implemented method of any of claims 1-3, further comprising:
determining a confidence metric measuring confidence associated with an assignment of the one or more identified additional individuals to the parental side of the target individual.

5. The computer-implemented method of any of claims 1-4, wherein each matched segment overlaps with a corresponding segment of the target individual by more than a predetermined threshold amount of sequence overlap.

6. The computer-implemented method of any of claims 1-5, wherein linking one or more first parental groups and one or more second parental groups across the plurality of sub-cluster pairs is based on similarities among the parental groups across the plurality of sub-cluster pairs, the similarities based on a number of common additional datasets classified in different parental groups across the plurality of sub-cluster pairs.

7. The computer-implemented method of any of claims 1-6, wherein linking one or more first parental groups and one or more second parental groups across the plurality of sub-cluster pairs is based on a heuristic scoring approach measuring similarities among the parental groups across the plurality of sub-cluster pairs.

8. The computer-implemented method of any of claims 1-7, wherein linking one or more first parental groups and one or more second parental groups across the plurality of sub-cluster pairs is based on a bipartite graph that matches different sub-cluster and sub-parent combinations.

9. The computer-implemented method of any of claims 1-8, further comprising correcting a genotyping error or a haplotype phasing error in the target individual genotype dataset.

10. The computer-implemented method of any of claims 1-9, wherein at least one of the match segments is identified by:
identifying a candidate match segment of one of the additional individual genotype datasets that matches a corresponding segment of the target individual genotype dataset;
dividing the candidate match segment into a plurality of sites;
determining a length of the plurality of sites that are classified to the parental side; and
determining, responsive to the length exceeding a threshold, that the plurality of sites are the at least one of the match segments.

11. The computer-implemented method of any of claims 1-10, wherein the super-cluster is a first super-cluster and the parental side is a first parental side, and the method further comprises:
identifying a second super-cluster for a second parental side;
identifying one or more additional individuals whose additional individual genotype datasets are classified to both the first and second super-clusters; and
removing the identified additional individuals from being associated with the first super-cluster or the second super-cluster.

12. The computer-implemented method of any of claims 1-11, further comprising:
identifying a first informative site, the informative site being a site with heterozygous allele values in the target individual genotype dataset and a homozygous allele value in a first additional individual genotype dataset;
classifying a first candidate matched segment of the first additional individual genotype dataset to a first parental group;
identifying a second additional individual genotype dataset also having the homozygous allele value at the first informative site;
classifying a second candidate matched segment of the second individual genotype dataset to the first parental group;
continuing to examine the target individual genotype dataset at a second informative site;
determining that the first individual genotype dataset and the second individual genotype dataset have contradicting homozygous alleles at the second informative site, the second informative site being a conflicting site; and
breaking the second candidate matched segment into two segments to separate the conflicting site.

13. The computer-implemented method of claim 12, further comprising:
repeating classifications of a plurality of candidate matched segments of other additional individual genotype datasets based on one or more informative sites, the classifications assigning the plurality of candidate matched segments to the first parental group and a second parental group.

14. A non-transitory computer readable medium storing computer code comprising instructions, when executed by one or more processors, causing the one or more processors to perform a method according to any one of the preceding claims.

15. A system comprising:
one or more processors; and
memory storing computer code comprising instructions, when executed by the one or more processors, causing the one or more processors to perform a method according to any one of claims 1-13.

## Patentansprüche

1. Computerimplementiertes Verfahren, umfassend:
Empfangen eines Zielindividuum-Genotypdatensatzes eines Zielindividuums;
Identifizieren einer Vielzahl von abgestimmten Segmenten (420) von Identität nach Abstammung (IBD), wobei jedes abgestimmte Segment zwischen dem Zielindividuum und einem zusätzlichen Individuum nach Identität nach Abstammung (IBD) gemeinsam ist, wobei Identifizieren der Vielzahl von abgestimmten Segmenten Vergleichen einer Vielzahl von Genotypdatensätzen des zusätzlichen Individuums mit dem Genotypdatensatz des Zielindividuums umfasst;
Identifizieren der abgestimmten Segmente für die zusätzlichen Individuen, die eine Länge von IBD-Segmenten mit dem Zielindividuums gemeinsam haben;
Klassifizieren der identifizierten abgestimmten Segmente in eine Vielzahl von Subcluster-Paaren (410), wobei jedes Subcluster-Paar einen Genlocus in einer Vielzahl von Genloci darstellt, und wobei jedes Subcluster-Paar eine erste Elterngruppe aufweist, die einen ersten Satz abgestimmter Segmente umfasst, und eine zweite Elterngruppe, die einen zweiten Satz abgestimmter Segmente umfasst;
Erzeugen eines Superclusters (460) einer Elternseite durch Verknüpfen von einer oder mehren ersten Elterngruppen und von einer oder mehreren zweiten Elterngruppen über die Vielzahl von Subcluster-Paaren, wobei Erzeugen des Superclusters der Elternseite Folgendes umfasst:
Definieren einer Kandidatenkonfiguration für den Supercluster, wobei die Kandidatenkonfiguration Kandidaten für Elternseitenzuweisungen der Elterngruppen über die Vielzahl von Subcluster-Paaren umfasst,
Bestimmen einer Anzahl von gemeinsamen Genotypdatensätzen des zusätzlichen Individuums,
die in die Kandidatenkonfiguration klassifiziert sind,
Umstellen der Kandidaten für Elternseitenzuweisungen, um die Anzahl gemeinsamer Genotypdatensätzen des zusätzlichen Individuums, die in die Kandidatenkonfiguration klassifiziert sind, zu erhöhen; und
Erzeugen einer Haplotyp-Phasierung des Zielindividuums aus dem Supercluster der Elternseite.

2. Computerimplementiertes Verfahren nach Anspruch 1, wobei das Klassifizieren der identifizierten abgestimmten Segmente in eine Vielzahl von Subcluster-Paaren Folgendes umfasst:
Identifizieren einer heterozygoten Allelstelle im Genotypdatensatz des Zielindividuums, wobei der Genotypdatensatz des Zielindividuums ein erstes Allel und ein zweites Allel an der heterozygoten Allelstelle aufweist,
Klassifizieren der abgestimmten Segmente, die eine erste entsprechende Stelle aufweisen, die das erste Allel aufweist und homozygot ist, zu der ersten Elterngruppe, und
Klassifizieren der abgestimmten Segmente, die eine zweite entsprechende Stelle aufweisen, die das zweite Allel aufweist und homozygot ist, zu der zweite Elterngruppe.

3. Computerimplementiertes Verfahren nach Anspruch 1 oder Anspruch 2, weiter umfassend:
Bestimmen der Elternseite als entweder väterlich oder mütterlich anhand eines oder mehrerer der folgenden Merkmale:
Zugreifen auf genealogische Daten des Zielindividuums, um mindestens ein Individuum in den genealogischen Daten zu identifizieren, das einer Elternseite zugeordnet ist, wobei gemäß den genealogischen Daten das mindestens eine identifizierte Individuum entweder der väterlichen oder der mütterlichen Seite des Zielindividuums angehört,
Übertragen iner Anfrage an einen Benutzer bezüglich einer Beziehung zwischen dem Zielindividuum und einem der identifizierten zusätzlichen Individuen, die einer Elternseite zugeordnet sind,
Untersuchen eines Genlocus von Geschlechtschromosomen in der Elternseite, um zu bestimmen, ob die Elternseite väterlich oder mütterlich ist,
Untersuchen eines Genlocus von Mitochondrien-DNA in der Elternseite, um zu bestimmen, ob die Elternseite väterlich oder mütterlich ist,
Bestimmen einer Ethnizität von einer oder mehreren identifizierten zusätzlichen Individuen, die zur Elternseite gehören, und/oder
Übertragen an einen Benutzer einer Anfrage über eine genetische Gemeinschaft, der das Zielindividuum angehört.

4. Computerimplementiertes Verfahren nach einem der Ansprüche 1-3, weiter umfassend: Bestimmen einer Konfidenzwerts, der die einer Zuweisung des einen oder der mehreren identifizierten zusätzlichen Individuen zur Elternseite des Zielindividuums zugeordneten Konfidenz misst.

5. Computerimplementiertes Verfahren nach einem der Ansprüche 1-4, wobei jedes abgestimmte Segment ein entsprechendes Segment des Zielindividuums um mehr als ein vorbestimmte Schwellenwertmaß von Sequenzüberlappung überlappt.

6. Computerimplementiertes Verfahren nach einem der Ansprüche 1-5, wobei das Verknüpfen einer oder mehrerer erster Elterngruppen mit einer oder mehrerer zweiter Elterngruppen auf Ähnlichkeiten zwischen den Elterngruppen über die Vielzahl von Subcluster-Paaren basiert, wobei die Ähnlichkeiten auf der Anzahl gemeinsamer zusätzlicher Datensätze beruhen, die über die Vielzahl von Subcluster-Paaren unterschiedlichen Elterngruppen zugeordnet sind.

7. Computerimplementiertes Verfahren nach einem der Ansprüche 1-6, wobei das Verknüpfen einer oder mehrerer erster Elterngruppen mit einer oder mehrerer zweiter Elterngruppen auf der Vielzahl von Subcluster-Paaren basiert, wobei ein heuristischer Punktwertansatz verwendet wird, der die Ähnlichkeiten zwischen den Elterngruppen anhand der Vielzahl von Subcluster-Paaren misst.

8. Computerimplementiertes Verfahren nach einem der Ansprüche 1-7, wobei das Verknüpfen einer oder mehrerer erster Elterngruppen mit einer oder mehrerer zweiter Elterngruppen auf der Vielzahl von Subcluster-Paaren basiert, die auf einem bipartiten Graphen beruhen, welcher unterschiedliche Untercluster und Elternunterkombinationen abstimmt.

9. Computerimplementiertes Verfahren nach einem der Ansprüche 1-8, weiter umfassend Korrigieren eines Genotypisierungsfehlers oder eines Haplotyp-Phasierungsfehlers in dem Genotypdatensatz des Zielindividuums.

10. Computerimplementiertes Verfahren nach einem der Ansprüche 1-9, wobei mindestens eines der Übereinstimmungssegmente identifiziert wird durch:
Identifizieren eines Kandidaten für ein Übereinstimmungssegment aus einem der Genotypdatensätze des zusätzlichen Individuums, das mit einem entsprechenden Segment des Genotypdatensatzes des Zielindividuums übereinstimmt;
Teilen des Kandidaten für ein Übereinstimmungssegment in eine Vielzahl von Stellen;
Bestimmen einer Länge der Vielzahl von Stellen, die zu der Elternseite klassifiziert sind; und
Bestimmen, als Reaktion darauf, dass die Länge einen Schwellenwert übersteigt, dass die Vielzahl von Stellen das mindestens eine der Übereinstimmungssegmente ist.

11. Computerimplementiertes Verfahren nach einem der Ansprüche 1-10, wobei der Supercluster ein erster Supercluster und die Elternseite eine erste Elternseite ist, und wobei das Verfahren weiter Folgendes umfasst:
Identifizieren eines zweiten Superclusters für eine zweite Elternseite;
Identifizieren einer oder mehrerer zusätzlicher Individuen, deren Genotypdatensätze des zusätzlichen Individuums sowohl zum ersten als auch zum zweiten Supercluster klassifiziert sind; und
Entfernen der identifizierten zusätzlichen Individuen aus der Zuweisung zu dem ersten Supercluster oder dem zweiten Supercluster.

12. Computerimplementiertes Verfahren nach einem der Ansprüche 1-11, weiter umfassend:
Identifizieren einer ersten informativen Stelle, wobei die informative Stelle eine Stelle mit heterozygoten Allelwerten im Genotypdatensatz des Zielindividuums und einem homozygoten Allelwert im Genotypdatensatz eines ersten zusätzlichen Individuums ist;
Klassifizieren eines ersten Kandidaten für ein abgestimmtes Segment des Genotypdatensatzes des ersten zusätzlichen Individuums zu einer ersten Elterngruppe;
Identifizieren des Genotypdatensatzes eines zweiten zusätzlichen Individuums, das auch den homozygoten Allelwert an der ersten informativen Stelle aufweist;
Klassifizieren eines zweiten Kandidaten für ein abgestimmtes Segment des Genotypdatensatzes des zweiten zusätzlichen Individuums zu der ersten Elterngruppe;
Fortführen der Untersuchung des Genotypdatensatzes des Zielindividuums an einer zweiten informativen Stelle;
Bestimmen, dass der Genotypdatensatz des ersten Individuums und der Genotypdatensatz des zweiten Individuums widersprüchliche homozygote Allele an der zweiten informativen Stelle aufweisen, wobei die zweite informative Stelle eine Konfliktstelle ist; und
Aufbrechen des zweiten Kandidaten für ein abgestimmtes Segment in zwei Segmente, um die Konfliktstelle abzutrennen.

13. Computerimplementiertes Verfahren nach Anspruch 12, weiter umfassend:
Wiederholen der Klassifizierungen einer Vielzahl von Kandidaten für abgestimmte Segmente aus anderen Genotypdatensätzen zusätzlicher Individuen auf Grundlage einer oder mehrerer informativer Stellen, wobei die Klassifizierungen die Vielzahl von Kandidaten entweder der ersten Elterngruppe oder der zweiten Elterngruppe zuordnen

14. Nicht-flüchtiges computerlesbares Medium, das Computercode speichert, der Anweisungen umfasst, die, wenn sie von einem oder mehreren Prozessoren ausgeführt werden, bewirken, dass der eine oder die mehreren Prozessoren ein Verfahren gemäß einem der vorstehenden Ansprüche durchführen.

15. System, umfassend:
einen oder mehrere Prozessoren; und
Speicher, der dem Computercode gespeichert ist, der Anweisungen umfasst, die, wenn sie von dem einen oder den mehreren Prozessoren ausgeführt werden, bewirken, dass der eine oder die mehreren Prozessoren ein Verfahren nach einem der Ansprüche 1-13 durchführen.

## Revendications

1. Procédé mis en œuvre par ordinateur comprenant :
la réception d'un ensemble de données de génotype d'individu cible d'un individu cible ;
l'identification d'une pluralité de segments adaptés d'identité par descendance (IBD) (420), dans lequel chaque segment adapté est partagé entre l'individu cible et un individu supplémentaire par identité par descendance (IBD), dans lequel l'identification de la pluralité de segments adaptés comprend la comparaison d'une pluralité d'ensembles de données de génotype d'individu supplémentaire avec l'ensemble de données de génotype d'individu cible ;
l'identification des segments adaptés pour les individus supplémentaires qui partagent une longueur de segments IBD avec l'individu cible ;
la classification des segments adaptés identifiés en une pluralité de paires de sous-groupes (410), dans lequel chaque paire de sous-groupes représente un locus génétique dans une pluralité de locus génétiques, et dans lequel chaque paire de sous-groupes présente un premier groupe parental comprenant un premier ensemble de segments adaptés et un second groupe parental comprenant un second ensemble de segments adaptés ;
la génération d'un super-groupe (460) d'un côté parental par liaison d'un ou plusieurs premiers groupes parentaux et d'un ou plusieurs seconds groupes parentaux à travers la pluralité de paires de sous-groupes, dans lequel la génération du super-groupe du côté parental comprend :
la définition d'une configuration candidate pour le super-groupe, la configuration candidate comprenant des attributions à un côté parental candidat de groupes parentaux à travers la pluralité de paires de sous-groupes,
la détermination d'un nombre d'ensembles de données de génotype d'individu supplémentaire communs qui sont classifiés dans la configuration candidate,
l'échange des attributions à un côté parental candidat pour augmenter un nombre d'ensembles de données de génotype d'individu supplémentaire communs qui sont classifiés dans la configuration candidate ; et
la génération d'une mise en phase d'haplotypes de l'individu cible à partir du super-groupe du côté parental.

2. Procédé mis en œuvre par ordinateur selon la revendication 1, dans lequel la classification des segments adaptés identifiés en une pluralité de paires de sous-groupes comprend :
l'identification d'un site d'allèle hétérozygote dans l'ensemble de données de génotype d'individu cible, l'ensemble de données de génotype d'individu cible présentant un premier allèle et un second allèle au niveau du site d'allèle hétérozygote,
la classification des segments adaptés présentant un premier site correspondant qui présente le premier allèle et est homozygote au premier groupe parental, et
la classification des segments adaptés présentant un second site correspondant qui présente le second allèle et est homozygote au second groupe parental.

3. Procédé mis en œuvre par ordinateur selon la revendication 1 ou la revendication 2, comprenant en outre :
l'identification du côté parental comme soit paternel soit maternel par une ou plusieurs des opérations suivantes :
l'accès à des données généalogiques de l'individu cible pour identifier au moins un individu dans les données généalogiques qui appartiennent au côté parental, le au moins un individu identifié appartenant soit à un côté paternel soit à un côté maternel de l'individu cible selon les données généalogiques,
la transmission, à un utilisateur, d'une interrogation concernant une relation entre l'individu cible et l'un des individus supplémentaires identifiés appartenant au côté parental,
l'examen d'un locus génétique de chromosomes sexuels dans le côté parental pour déterminer si le côté parental est paternel ou maternel,
l'examen d'un locus génétique d'ADN mitochondrial dans le côté parental pour déterminer si le côté parental est paternel ou maternel,
la détermination d'une ethnicité d'un ou plusieurs individus supplémentaires identifiés appartenant au côté parental, et/ou
la transmission, à un utilisateur, d'une interrogation concernant une communauté génétique à laquelle l'individu cible appartient.

4. Procédé mis en œuvre par ordinateur selon l'une quelconque des revendications 1 à 3, comprenant en outre : la détermination d'une métrique de confiance mesurant une confiance associée à une attribution des un ou plusieurs individus supplémentaires identifiés au côté paternel de l'individu cible.

5. Procédé mis en œuvre par ordinateur selon l'une quelconque des revendications 1 à 4, dans lequel chaque segment adapté chevauche un segment correspondant de l'individu cible de plus d'une quantité de seuil prédéterminée de chevauchement de séquence.

6. Procédé mis en œuvre par ordinateur selon l'une quelconque des revendications 1 à 5, dans lequel la liaison d'un ou plusieurs premiers groupes parentaux et d'un ou plusieurs seconds groupes parentaux à travers la pluralité de paires de sous-groupes est basée sur des similitudes parmi les groupes parentaux à travers la pluralité de paires de sous-groupes, les similitudes étant basées sur un nombre d'ensembles de données supplémentaires communs classifiés dans des groupes parentaux différents à travers la pluralité de paires de sous-groupes.

7. Procédé mis en œuvre par ordinateur selon l'une quelconque des revendications 1 à 6, dans lequel la liaison d'un ou plusieurs premiers groupes parentaux et d'un ou plusieurs seconds groupes parentaux à travers la pluralité de paires de sous-groupes est basée sur une approche de notation heuristique mesurant des similitudes parmi les groupes parentaux à travers la pluralité de paires de sous-groupes.

8. Procédé mis en œuvre par ordinateur selon l'une quelconque des revendications 1 à 7, dans lequel la liaison d'un ou plusieurs premiers groupes parentaux et d'un ou plusieurs seconds groupes parentaux à travers la pluralité de paires de sous-groupes est basée sur un graphe bipartite qui adapte différentes combinaisons de sous-groupes et de sous-parents.

9. Procédé mis en œuvre par ordinateur selon l'une quelconque des revendications 1 à 8, comprenant en outre la correction d'une erreur de génotypage ou d'une erreur de mise en phase d'haplotypes dans l'ensemble de données de génotype d'individu cible.

10. Procédé mis en œuvre par ordinateur selon l'une quelconque des revendications 1 à 9, dans lequel au moins l'un des segments adaptés est identifié par :
l'identification d'un segment adapté candidat de l'un des ensembles de données de génotype d'individu supplémentaire qui est adapté à un segment correspondant de l'ensemble de données de génotype d'individu cible ;
la division du segment adapté candidat en une pluralité de sites ;
la détermination d'une longueur de la pluralité de sites qui sont classifiés dans le côté parental ; et
la détermination, en réponse au dépassement d'un seuil par la longueur, du fait que la pluralité de sites sont le au moins un des segments adaptés.

11. Procédé mis en œuvre par ordinateur selon l'une quelconque des revendications 1 à 10, dans lequel le super-groupe est un premier super-groupe et le côté parental est un premier côté parental, et le procédé comprend en outre :
l'identification d'un second super-groupe pour un second côté parental ;
l'identification d'un ou plusieurs individus supplémentaires dont des ensembles de données de génotype d'individu supplémentaire sont classifiés à la fois dans les premier et second super-groupes ; et
le retrait des individus supplémentaires identifiés de l'association avec le premier super-groupe ou le second super-groupe.

12. Procédé mis en œuvre par ordinateur selon l'une quelconque des revendications 1 à 11, comprenant en outre :
l'identification d'un premier site d'informations, le site d'informations étant un site avec des valeurs d'allèle hétérozygote dans l'ensemble de données de génotype d'individu cible et une valeur d'allèle homozygote dans un premier ensemble de données de génotype d'individu supplémentaire ;
la classification d'un premier segment adapté candidat du premier ensemble de données de génotype d'individu supplémentaire dans un premier groupe parental ;
l'identification d'un second ensemble de données de génotype d'individu supplémentaire présentant également la valeur d'allèle homozygote au niveau du premier site d'informations ;
la classification d'un second segment adapté candidat du second ensemble de données de génotype d'individu dans le premier groupe parental ;
la continuation de l'examen de l'ensemble de données de génotype d'individu cible au niveau d'un second site d'informations ;
la détermination du fait que le premier ensemble de données de génotype d'individu et le second ensemble de données de génotype d'individu présentent des allèles homozygotes contradictoires au niveau du second site d'informations, le second site d'informations étant un site conflictuel ; et
la rupture du second segment adapté candidat en deux segments pour séparer le site conflictuel.

13. Procédé mis en œuvre par ordinateur selon la revendication 12, comprenant en outre :
la répétition de classifications d'une pluralité de segments adaptés candidats d'autres ensembles de données de génotype d'individu supplémentaire sur la base d'un ou plusieurs sites d'informations, les classifications attribuant la pluralité de segments adaptés candidats au premier groupe parental et à un second groupe parental.

14. Support non transitoire lisible par ordinateur stockant un code d'ordinateur comprenant des instructions, lorsqu'elles sont exécutées par un ou plusieurs processeurs, amenant les un ou plusieurs processeurs à réaliser un procédé selon l'une quelconque des revendications précédentes.

15. Système comprenant :
un ou plusieurs processeurs ; et
une mémoire stockant un code d'ordinateur comprenant des instructions, lorsqu'elles sont exécutées par les un ou plusieurs processeurs, amenant les un ou plusieurs processeurs à réaliser un procédé selon l'une quelconque des revendications 1 à 13.
